(19) Europäisches Patentamt
European Patent Office
Office européen des brevets

(11) **EP 2 374 016 B1**

(12) **EUROPEAN PATENT SPECIFICATION**

(45) Date of publication and mention of the grant of the patent:
**10.05.2017 Bulletin 2017/19**

(51) Int Cl.:
*G01R 33/28* (2006.01)   *G01R 33/46* (2006.01)
*A61K 49/06* (2006.01)   *C07F 15/00* (2006.01)
*A61K 49/10* (2006.01)   *G01R 33/56* (2006.01)

(21) Application number: **09801523.3**

(22) Date of filing: **10.12.2009**

(86) International application number:
**PCT/GB2009/002860**

(87) International publication number:
**WO 2010/067076 (17.06.2010 Gazette 2010/24)**

(54) **PULSE SEQUENCING WITH HYPERPOLARISABLE NUCLEI**

IMPULSSEQUENZIERUNG MIT HYPERPOLARISIERBAREN KERNEN

SEQUENCAGE D'IMPULSIONS A NOYAUX HYPERPOLARISABLES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.12.2008 GB 0822484**
**11.03.2009 GB 0904201**

(43) Date of publication of application:
**12.10.2011 Bulletin 2011/41**

(73) Proprietor: **University of York**
**York YO10 5DD (GB)**

(72) Inventors:
• **DUCKETT, Simon, Benedict**
**York YO10 3KR (GB)**
• **GREEN, Gary, George, Reginald**
**Benton NE7 7XE (GB)**
• **COWLEY, Michael, James**
**West Sussex RH16 3SJ (GB)**

(74) Representative: **Gilholm, Stephen Philip**
**IPheions Intellectual Property**
**Buzzard Office**
**The Hawk Creative Business Park**
**Easingwold,**
**York YO61 3FE (GB)**

(56) References cited:
• **BOUCHARD L-S ET AL: "Para-Hydrogen-Enhanced Hyperpolarized Gas-Phase Magnetic Resonance Imaging" ANGEWANDTE CHEMIE. INTERNATIONAL EDITION, WILEY VCH VERLAG, WEINHEIM LNKD- DOI:10.1002/ANIE.200700830, vol. 46, no. 22, 23 April 2007 (2007-04-23), pages 4064-4068, XP002497396 ISSN: 1433-7851**
• **GRANT A K ET AL: "Early Experience with Simple Methods for Parahydrogen-Induced Hyperpolarization" INTERNATIONAL SOCIETY FOR MAGNETIC RESONANCE IN MEDICINE.SCIENTIFIC MEETING AND EXHIBITION. PROCEEDINGS, INTERNATIONAL SOCIETY FOR MAGNETIC RESONANCE IN MEDICINE, US, 6 May 2006 (2006-05-06), page 2552, XP002497397 ISSN: 1524-6965**
• **BARGON J ET AL: "Parahydrogen-Induced Hyperpolarization of 15N" INTERNATIONAL SOCIETY FOR MAGNETIC RESONANCE IN MEDICINE.SCIENTIFIC MEETING AND EXHIBITION. PROCEEDINGS, INTERNATIONAL SOCIETY FOR MAGNETIC RESONANCE IN MEDICINE, US, 19 May 2007 (2007-05-19), page 1317, XP002497398 ISSN: 1524-6965**
• **BLAZINA D ET AL: "Applications of the parahydrogen phenomenon in inorganic chemistry" DALTON TRANSACTIONS, RSC PUBLISHING, CAMBRIDGE, GB LNKD-DOI:10.1039/B409606A, no. 17, 7 September 2004 (2004-09-07), pages 2601-2609, XP002497399 ISSN: 1477-9226 [retrieved on 2004-08-05]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- AGUILAR JUAN A ET AL: "Only para-hydrogen spectroscopy (OPSY), a technique for the selective observation of para-hydrogen enhanced NMR signals." CHEMICAL COMMUNICATIONS (CAMBRIDGE, ENGLAND) 12 MAR 2007 LNKD- PUBMED:17347733, no. 11, 12 March 2007 (2007-03-12), pages 1183-1185, XP002576966 ISSN: 1359-7345
- GNANAMGARI D ET AL: "Transfer hydrogenation of imines and alkenes and direct reductive amination of aldehydes catalyzed by triazole-derived iridium(I) carbene complexes" ORGANOMETALLICS 20070226 AMERICAN CHEMICAL SOCIETY US LNKD- DOI:10.1021/OM060938M, vol. 26, no. 5, 26 February 2007 (2007-02-26), pages 1226-1230, XP002576967
- ADAMS R W ET AL: "Reversible interactions with para-hydrogen enhance NMR sensitivity by polarization transfer" SCIENCE AMERICAN ASSOCIATION FOR THE ADVANCEMENT OF SCIENCE USA LNKD- DOI:10.1126/SCIENCE.1168877, vol. 323, no. 5922, 27 March 2009 (2009-03-27), pages 1708-1711, XP002576968 ISSN: 0036-8075
- ADAMS R W ET AL: "A theoretical basis for spontaneous polarization transfer in non-hydrogenative parahydrogen-induced polarization" JOURNAL OF CHEMICAL PHYSICS, AMERICAN INSTITUTE OF PHYSICS, NEW YORK, NY, US LNKD- DOI:10.1063/1.3254386, vol. 131, no. 19, 16 November 2009 (2009-11-16), pages 194505/1-15, XP009131835 ISSN: 0021-9606
- ATKINSON KEVIN D ET AL: "Para-hydrogen induced polarization without incorporation of para-hydrogen into the analyte." INORGANIC CHEMISTRY 19 JAN 2009 LNKD- PUBMED:19072592, vol. 48, no. 2, 19 January 2009 (2009-01-19), pages 663-670, XP002576969 ISSN: 1520-510X

**Description**

**Field of the invention**

The present invention relates to a method of selective observation of non-hydrogenative *para*-hydrogen induced polarisation (NH-PHIP) as enhanced magnetic resonance signals which comprise thermal and longitudinal spin order states.

**Background of the invention**

Our co-pending International Patent application No. WO2008/155093 describes a method for carrying out an NMR experiment with enhanced sensitivity on a compound comprising hyperpolarisable nuclei, with the steps of:

a) preparing a fluid having a temperature TF, containing spatially symmetric molecules comprising two halves each, with a non-Boltzmann nuclear spin state distribution of the symmetric molecules at this temperature TF;
b) providing a compound with a defined chemical identity;
c) providing a template that offers sites of ordered environment for the two halves of a symmetric molecule and a compound which can be arranged at each site, wherein the ordered environment distinguishes chemically or magnetically the two halves of a symmetric molecule arranged at each site, and wherein the ordered environment allows interaction via scalar coupling or dipolar coupling between the two halves of a symmetric molecule and a compound arranged at each site;
d) bringing together the prepared fluid, the provided compound and the provided template, thereby transferring the spin order from the symmetric molecules to the hyperpolarisable nuclei of the compound during a temporary association of the symmetric molecules, the compound, and the template while ultimately keeping the chemical identity of the compound in an appropriately sized magnetic field; and
e) performing an NMR measurement on the compound comprising hyperpolarized nuclei prepared in step d).

Also our co-pending and as yet unpublished, Patent application No. GB 0822484.2 describes a method of selective observation of non-hydrogenative *para*-hydrogen induced polarisation (NH-PHIP) as enhanced magnetic resonance signals.

NMR and MRI involve the detection of what can be viewed to be transitions of nuclear spins between an excited state and a ground state in an applied magnetic field. Because the energy difference between these states is relatively small, the usual Boltzmann distribution of chemically identical nuclei is such that at room temperature the populations of nuclear spin states which are in dynamic equilibrium are almost identical. Since the strength of the detected signal in magnetic resonance experiments is proportional to the population difference, NMR and MRI signals are typically weak.

The strength of detectable NMR signals can however be enhanced by hyperpolarizing the magnetic nuclei. Hyperpolarisation in this context refers to a process in which a significant excess of magnetic nuclei are induced into the same spin state. This results in a large increase in available signal due to the much larger inequality of populations across the energy levels. In order for a hyperpolarized state to be useful, it is important that the spin state is sufficiently long lived to provide useful information, i.e. that the relaxation time of the spin state is 'long'. The rules governing the relaxation rates of nuclear spins are complex but known. It suffices to say that certain nuclei and spins systems have relaxation times which may extend from seconds to hours, days, months or even years.

There are a number of ways to induce certain nuclei into a hyperpolarized state. The simplest way is to cool the material to very low temperatures in the presence of a magnetic field, which will favour population of the lower energy state in which the spins of the nuclei are aligned with the applied magnetic field. This method is suitable for the production of hyperpolarized monatomic gases such as xenon or helium-3. The polarization levels of these nuclei have also been increased via the use of laser-based technologies.

One molecule that can be readily polarised is dihydrogen. Dihydrogen exists in various spin states, in which the spins of the individual nuclei are either aligned (ortho, the higher energy state), or opposed (para, the lower energy spin state). Para-hydrogen ($p$-$H_2$) is a nuclear spin isomer of dihydrogen with the spin configuration $\alpha\beta$-$\beta\alpha$. Para-hydrogen has no net magnetic moment and is therefore unobservable in this form by magnetic resonance methods. The ortho forms however retain magnetic resonance activity. The binuclear spin system of dihydrogen can be converted into parahydrogen simply by cooling to low temperature in the presence of a suitable catalyst which promotes conversion to the lower energy para-hydrogen state. In this process, the role of the catalyst is to perturb the dihydrogen molecule and thereby reduce its symmetry; otherwise a quantum mechanical selection rule prevents interconversion between the two spin states. Once separated from the catalyst and returned to room temperature, the para-hydrogen spin state may last for over a year in the absence of external effects. This form of hydrogen corresponds to a singlet state.

Nuclei can be hyperpolarized by a process known as para-hydrogen induced polarization (PHIP). PHIP has proved to be highly efficient and has currently achieved greater enhancement of heteronuclei NMR signals than other methods known in the art. PHIP is generally the result of a chemical reaction in which the para-hydrogen nuclei are transferred irreversibly into another molecule having certain symmetry properties. Under the right circumstances, the spin state of the para-hydrogen molecule is preserved in the spins of the two hydrogen atoms which become part of the new molecule. If other NMR-active nuclei are within coupling distance of the hydrogen nuclei, spin polarization of those nuclei can be transferred spontaneously in an optimal magnetic field. In this way, the signals of heteronuclei such as $^{13}C$, $^{15}N$ and $^{31}P$ can be enhanced. By way of example, WO 99/24080 describes a PHIP process in which para-hydrogen is added across a symmetrical carbon-carbon double bond containing a $^{13}C$ centre. In one example of such a process, Wilkinson's catalyst is first reduced by addition of para-hydrogen, followed by addition of an ethylene ligand. The resulting hydride ligands then undergo a migratory insertion reaction with the ethylene ligand, which subsequently dissociates from the complex to form uncoordinated hyperpolarized ethane. An overview of PHIP is given in Blazina et al, Dalton Trans., 2004, 2601-2609.

Conventional PHIP processes therefore involve the chemical addition of para-hydrogen to hydrogenatable substrates (compounds), usually organic substrates (compounds) containing double and triple bonds. These processes are therefore limited to substrates (compounds) capable of undergoing hydrogenation. Furthermore, hydrogen equivalence is not preserved at all stages, which leads to some loss of hyperpolarisation through relaxation.

Only *Para* Hydrogen Spectroscopy (OPSY) is a technique for the selective observation of para-hydrogen enhanced NMR signals in such hydrogenation products and works by filtering thermal signals from, *inter alia,* $^{1}H$ NMR spectra.

We now describe a series of related patents that illustrate some of the potential application of hyperpolarised magnetic resonance methods.

European Patent application No. EP1047455 and US Patent No. 6,574,495 describe a method of magnetic resonance investigation of a sample which comprises reacting para-hydrogen enriched hydrogen with a hydrogenatable MR imaging agent precursor containing a non-hydrogen non zero nuclear spin nucleus to produce a hydrogenated MR imaging agent; administering the hydrogenated MR imaging agent to the sample; exposing the sample to radiation of a frequency selected to excite nuclear spin transitions of the non-zero nuclear spin nucleus in the hydrogenated MR imaging agent; and detecting magnetic resonance signals of the non-zero nuclear spin nucleus from the sample.

US Patent application No. US 2004/0024307 describes the use of para-hydrogen enriched hydrogen wherein the proportion of para-hydrogen is more than 45% in the manufacture of an MR imaging agent and wherein the MR imaging agent is produced by reaction of a MR imaging agent precursor containing one or more hydrogenatable unsaturated carbon-carbon bonds and a non-hydrogen non zero nuclear spin nucleus with said para-hydrogen enriched hydrogen for use in a method of diagnosis involving generation of an MR image by non-proton MR imaging.

International Patent application No. WO 2004/019995 describes an arrangement and a method for providing contrast agent for e.g. MRI and NMR applications. The method comprises the steps of obtaining a solution in a solvent of a hydrogenatable, unsaturated substrate compound and a catalyst for the hydrogenation of a substrate compound, hydrogenating the substrate with hydrogen gas ($H_2$) enriched in parahydrogen ($p$-$^{1}H_2$) to form a hydrogenated contrast agent and exposing the contrast agent to an oscillating magnetic field.

US Patent No. 7,459,144 describes a process for preparation of MR contrast agents by hydrogenation with o-deuterium or with p-hydrogen ($^{1}H_2$)/deuterium($^{2}H_2$) mixtures, in which the p/o ratio for hydrogen and the o/p ratio for deuterium are higher than the equilibrium values (1:3 and 2:1) at ambient temperature.

These patents therefore illustrate how hydrogenative methods provide a route with parahydrogen to hydrogenise a substrate and then to image it.

International Patent application No. WO 99/35508 and European Patent application No. EP1046051 describe a method of magnetic resonance investigation of a sample comprising producing a hyperpolarised solution of a high T agent, wherein the high T agent in the hyperpolarised solution has a T1 value (at a field strength in the range 0.01-5T and a temperature in the range 20-40°C) of at least 5 seconds, by dissolving a hyperpolarised solid sample in a solvent where the hyperpolarisation of the solid sample is effected by means of a polarising agent, separating the polarising agent from the high T agent administering the hyperpolarised solution to the sample exposing the sample to a second radiation of a frequency selected to excite nuclear spin transitions in the MR imaging nuclei of the high T1 agent; detecting magnetic resonance signals from the sample; and generating an image, dynamic flow data, diffusion data, perfusion data, physiological data or metabolic data from the detected signals.

These patents therefore illustrate the potential of hyperpolarisation in imaging and the examination of labelled material via a DNP (Dynamic Nuclear Polarisation) polarisation route.

US Patent No. 6,311,086 describes a method of MR investigation of a sample comprising placing in a uniform magnetic field a composition comprising an Overhauser magnetic resonance imaging (OMRI) contrast agent and an MR imaging agent containing in its molecular structure nuclei capable of emitting magnetic resonance signals and having a T1 relaxation time of 6s or more (at 37°C in $D_2O$ in a field of 7T); exposing the composition to a radiation of a frequency selected to excite electron spin transitions in the OMRI contrast agent and thereby cause a nuclear spin polarisation of the nuclei; separating the MR imaging agent from the OMRI contrast agent; administering the MR imaging agent to the sample; exposing the sample to a second radiation of a frequency selected to excite nuclear spin transitions; and detecting magnetic resonance signals from the sample. This patent illustrates a different route to hyperpolarisation.

US Patent No. 6,466,814 describes a method of MR investigation of a sample comprising producing a hyperpolarised solution of a high T1 agent by dissolving a hyperpolarised solid sample of the high T1 agent in a solvent; where the hyperpolarisation of the solid sample of the high T1 agent is effected by means of a polarising agent, separating the polarising agent from the high T1 agent; administering the hyperpolarised solution to the sample; exposing the sample to radiation of a frequency selected to excite nuclear spin transitions in an MR imaging nuclei of the high T1 agent; detecting magnetic resonance signals from the sample; wherein the high T1 agent in the hyperpolarised solution has a T1 value (at a field strength in the range 0.01-5 T and a temperature in the range 20-40°C) of at least 5 seconds and wherein the high T1 agent is $^{13}$C enriched at one or more carbonyl or quaternary carbon positions. This patent illustrates how labelled materials can be hyperpolarised using a different method.

US Patent application No. 2004/0024307 describes a method of MR investigation of a sample, the method comprising reacting para-hydrogen enriched hydrogen, in which the enriched hydrogen has a more than 45% proportion of para-hydrogen with a hydrogenatable MR imaging agent precursor containing a non-hydrogen non-zero nuclear spin nucleus to produce a hydrogenated MR imaging agent

US Patent No. 6,278,893 describes a method of MR investigation of a sample comprising subjecting a high T1 agent to *ex vivo* polarisation wherein the high T1 agent is a solid high T1 agent comprising nuclei selected from the group consisting of $^1$H, $^3$Li, $^{13}$C, $^{15}$N, $^{19}$F and $^{31}$P nuclei and wherein said solid high T1 agent is dissolved in an administrable media prior to administration to said sample.

International Patent application No. WO 02/23210 describes a method of MR investigation of a sample, comprising obtaining an MR imaging agent containing in its molecular structure at least one storage and one detection non-zero nuclear spin nuclei of different gyromagnetic ratio values wherein the storage and detection nuclei are present within the same molecule and wherein the storage and the detection nuclei are separated by from 2 to 5 chemical bonds. The application describes a DNP material, in particular, a contrast media containing hyperpolarised nuclei, such as $^{13}$C or $^{15}$N, which, when subjected to a pulse sequence transfers polarisation from the hyperpolarised $^{13}$C or $^{15}$N nuclei, to nuclei having a higher value of the gyromagnetic ratio, such as $^1$H, $^{19}$F or $^{31}$P.

International Patent application No. WO 02/23209 discloses novel deuterated compound and a method of contrast enhanced MR resonance imaging of a sample, the method comprising administering a hyperpolarised MR contrast agent comprising non-zero nuclear spin nuclei into the sample for fluid dynamic investigations of the vasculature.

International Patent application No. WO 2005/015253 describes an NMR method comprising providing a sample where the nuclear spin Hamiltonian operator of the component molecules of the sample possess one or more symmetry operations; creating a quasi equilibrium nuclear spin ensemble state in a sample, the quasi equilibrium nuclear spin ensemble state comprising at least two manifolds of spin states which transform differently under the symmetry operations of the Hamiltonian and the manifolds having different mean nuclear spin populations; allowing the quasi equilibrium nuclear spin ensemble state to remain for a time of equal to or substantially greater than 3TI, where T, is the spin lattice relaxation time; breaking a symmetry operation of the Hamiltonian; applying a sequence of magnetic fields to generate a nuclear magnetic resonance signal from the sample; and detecting the nuclear magnetic resonance signal.

The following three patents describe techniques of analysis. International Patent application No. WO 01/96895 describes a method for investigating the fate of a test compound containing an NMR active nuclei selected from $^{13}$C, $^{15}$N, $^{31}$P, $^1$H and $^{19}$F, which comprises administering the test compound to a biological system in which its fate is to be studied hyperpolarising the NMR active nuclei in the system and analysing the hyperpolarised system by NMR spectroscopy or NMR imaging in the liquid state.

US Patent application No. US 2006/0003926 describes novel compounds useful for the diagnosis and treatment of cancer and for monitoring therapeutic angiogenesis treatment and destruction of new angiogenic vasculature. The compounds disclosed therein are comprised of a targeting moiety that binds to a receptor that is upregulated during angiogenesis, an optional linking group, and a therapeutically effective radioisotope or diagnostically effective imageable moiety. The imageable moiety is a gamma ray or positron emitting radioisotope, a magnetic resonance imaging contrast agent, an X-ray contrast agent, or an ultrasound contrast agent.

International Patent application No. WO 2004/048988 describes a method for passive visualisation of invasive devices by employing a hyperpolarised solution of a high T1 agent having a T1 value of at least 5 seconds at a field strength in the range of 0.001-5 T and a temperature in the range of 20-40 °C.

International Patent application No. WO 2007136439 describes a method of producing a hyperpolarized material comprising a material selected from the group consisting of a liquid and a non noble gas at standard conditions and increasing the nuclear hyperpolarisation of the material until the material becomes hyperpolarized; and transferring the nuclear hyperpolarisation from the material to a second material.

International Patent application No. WO 2007/082048 describes the *ex vivo* induction of nuclear hyperpolarisation in imaging agents by temporarily shortening T1.

The majority of work described in these patents also resides in appropriate scientific journals and reference sources such as the Encyclopaedia of Magnetic Resonance. In the case of dynamic nuclear polarisation for example, a recent article by Kemsley, (Chemical and Engineering News 86, 12-15 (2008)) describes the state of the art. For parahydrogen, Duckett and Wood recently produced an article on parahydrogen-based NMR methods as a mechanistic probe in inorganic chemistry (Coordination Chemistry reviews. 2008, 252, 2278 - 2291).We have now found that applying thermal filtering techniques, such as OPSY with the non-hydrogenative para-hydrogen induced polarisation (NH-PHIP) that is achieved without any chemical change of the material as described earlier also allows thermal signals to be filtered or suppressed, whilst leaving those derived through non-hydrogenative *para*-hydrogen induced polarisation (PHIP) intact. Thus, this combined technique provides a new route for high sensitivity NMR measurements and MRI imaging measurements and can be employed as a key pre-observation building block for use with appropriately modified pulse sequences.

Rowlands, D. W. describes, in "Development of a Route to Spin-Polarized Metabolites by Polarization Transfer from Xenon-129" in the SURF Report [31 October 2007 *www.ugcs.caltech.edu/~**rowlands**/2nd**Surf**Report.pdf*] improved sensitivity and decreased measurement times in $^{13}$C NMR and MRI observation of small metabolites by the hyperpolarisation of the nuclear spins of $^{13}$C atoms in molecules by transferring hyperpolarisation from $^{129}$Xe by spin-exchange optical pumping, by direct dipolar couplings.

Bhattacharya, et al in "Ultra-fast three dimensional imaging of hyperpolarized 13C in vivo" MAGMA (2005) 18: 245-256; describes a chemical method of enhancing nuclear spin polarization of 13C. Two water soluble $^{13}$C imaging agents were hyperpolarized utilizing parahydrogen and an automated polarizer. $^{13}$C polarization was quantified in flow phantoms and in rats with jugular vein catheters. 3D-FIESTA was effective for sub-second *in vivo* imaging of hyperpolarized $^{13}$C reagents produced in a custom-built parahydrogen polarizer. Application to $^{13}$C hyperpolarized by parahydrogen was demonstrated *in vitro* and *in vivo.*

The article Bouchard L.-S. et al., Angew. Chem. Int. Ed., 2007, 46, 4064-4068, describes a method for carrying out an NMR experiment with enhanced sensitivity on a compound comprising hyperpolarizable nuclei, with the steps of preparing para-hydrogen with a non-Boltzmann nuclear spin state distribution of the symmetric molecules at the temperature of the fluid, providing a compound with a define chemical identiy, namely propylene, providing a template (Wilkinson's catalyst supported on modified silica gel) that offers sites of ordered environment for the two halves of para-hydrogen, wherein the ordered environment distinguishes chemically or magnetically the two haves of para-hydrogen, bringing together, at said temperature, the compound and the template, thereby transferring the spin order from para-hydrogen to the hyperpolarizable nuclei of the compound, and performing an NMR measurement on the compound comprising hyperpolarizable nuclei as prepared.

## Summary of the Invention

According to a first aspect of the invention we provide a method of selective observation of non-hydrogenative para-hydrogen induced polarisation (NH-PHIP) as enhanced magnetic resonance signals, said method comprises the steps of:

a) preparing a fluid containing spatially symmetric molecules comprising two halves each, with a non-Boltzmann nuclear spin state distribution of the symmetric molecules at the temperature of the fluid;
b) providing a compound with a defined chemical identity;
c) providing a template which binds or supports an interaction with parahydrogen and the compound and that offers sites of ordered environment for the two halves of the symmetric molecule and the compound which can be arranged at each site, wherein the ordered environment distinguishes chemically or magnetically the two halves of the symmetric molecule arranged at each site, and wherein the ordered environment allows interaction via scalar coupling or dipolar coupling between the two halves of the symmetric molecule and the compound arranged at each site; said template being selected from the group consisting of $[Ir(NHC)(H)_2(Py)_3]^+$, in which NHC is a saturated or unsaturated N-heterocyclic carbene; and analogues thereof;
d) bringing together, at said temperature, the prepared fluid, the compound and the assembled template, thereby transferring the spin order from the symmetric molecules to the hyperpolarisable nuclei of the compound during a temporary association of the symmetric molecules, the compound at the template while ultimately keeping the chemical identity of the compound;
e) performing an MR measurement on the compound comprising hyperpolarized nuclei prepared in step d); and
f) separating thermal and longitudinal spin order states by suppressing or filtering a thermal magnetisation background signal created due to the interaction with a magnetic field of an instrument that is used, by the use of appropriate pulse sequences made up of appropriately phased RF pulses and receiver phases.

It will be understood by the person skilled in the art that the thermal and longitudinal spin order states may not need to be completely separated, i.e. an unseparated mixture of thermal and longitudinal spin order states may still provide a significant enhancements in MRI.

The method of the invention observes the magnetic states generated through NH-PHIP and the thermal and longitudinal order states are separated by simultaneously, separately or sequentially suppressing or filtering a thermal background signal. We have also used the terms Signal Amplification by Reversible Exchange (SABRE) to refer to this effect.

It will be understood by the person skilled in the art that the method of the invention may be suitably utilised with short lived states, e.g. for use in connection with nuclear magnetic resonance (NMR) signals or rapid MRI measurements and for long lived states, e.g. for use in longer term magnetic resonance imaging (MRI) studies where chemical shift encoding enables a detailed view of metabolism or molecular processes to be assembled or analogous NMR measurements are completed.

When an inorganic template binds or supports an interaction with parahydrogen and a substrate, such that the two hydrogen atoms become distinct with regard to the substrate, polarisation transfer proceeds in low magnetic field to the magnetically active groups of the substrate. The type of magnetic states created in this process are controlled by the strength of low magnetic field which may lie, but need not be restricted to, between 0 and 1 T.

The creation of a long lived state through this approach means that it can be used to monitor metabolic pathways in real time and hence their response to treatment. In addition these signals can be used in a more conventional way to sensitise a generic substrate and then examine its perfusion within the body. The combination of these methods therefore provides a new route for diagnosis in MRI.

One important feature that needs to be recognised is that long lived states may be created for pairs (or higher values) of coupled spins. In the case where they are pairs, these could be $^1$H/$^1$H, $^1$H/$^{13}$C, $^1$H/$^{19}$F, $^1$H/$^{15}$N or $^{13}$C/$^{13}$C or any other combination of spin one half nuclei. In other words they can be either homo-nuclear or hetero-nuclear in nature. Indeed, it is a particular advantage of the present invention that the method may be used to observe either to proton, or what are commonly classed as hetero-nuclear spin states. There are therefore a range of options that are available to reading out this magnetisation which has been illustrated mainly for $^1$H but, it will be understood by a person skilled in the art that this approach will be equally valid for $^{13}$C, $^{15}$N etc.

The thermal and longitudinal order states are separated from each other by the use of appropriate pulse sequences. These can be made up of appropriately phased RF pulses and receiver phases and may include an element of magnetic field gradients which can be applied in one or several axes.

## Pulse sequence development

The thermal and longitudinal order states that are created by these methods are separated by the use of appropriate pulse sequences. These can be made up of appropriately phased RF pulses and receiver phases, and/or include an element of magnetic field gradients which can be applied in one or several axes.

One specific route to achieving this separation of parahydrogen derived signals from those of the background is based on exploiting differences that exist between the two types of magnetisation.

In practice this is can be achieved by using alternate addition and subtraction of $^1$H-NMR experiments such as those recorded using -45° and 135° pulse angles.

A second way to achieve this is by use of a material that may be enriched in a coupled heteronucleus, and a series of gradient pulses, such as that found in the traditional HMQC experiment. This approach allows the magnetisation to be selected based on the presence of the coupling to a second spin such as $^{13}$C or $^{31}$P. In this approach the second spin acts as a filter, by producing a linked magnetic state, which can be read directly or transferred to another spin, where it is detected.

A third way involves the use of a multiple quantum or coherence based filter. This might be called an *Only Para-Hydrogen SpectroscopY* (OPSY) approach. This method selects para-hydrogen hyperpolarised signals by exploiting pulse field gradients to select a coherence transfer pathway that leads only to the detection of signals of interest. As a one-dimensional technique it achieves selection in only one transient. Due to its ability to detect only hyperpolarised signals, however, the OPSY approach is also a background suppression technique that enables the real-time monitoring of *para*-hydrogen polarised products in protio rather than deuterio solvents.

The OPSY experiment works by taking term (1) for two spins that can be described in a longitudinal spin order state. We note that additional $I_{1x}I_{2x}$ and $I_{1y}I_{2y}$ terms are present in a singlet state.

$$p = I_{1z}I_{2z} \qquad\qquad (1)$$

A single $\pi/2$ *RF* pulse of phase *y* converts the term $I_{1z}I_{2z}$ into $I_{1x}I_{2x}$ while the same pulse converts the more usual thermal magnetisation present at the start into $I_{1z} + I_{2z}$ into $I_{1x} + I_{2x}$ terms. We note that an experienced worker will recognise these as example phases and angles since they can be varied or combined and yet still achieve the same net result.

Since the term $I_{1x}I_{2x}$ is a mixture of zero and double quantum coherences it is not directly observable but it can be converted into anti-phase magnetization using a second $\pi/2$ *RF* pulse provided there is an interpulse delay. In this way, $I_{1z}I_{2x}$ and $I_{1x}I_{2z}$ terms are created. By using appropriate coherence selection schemes, *para*-hydrogen signals that derive from the term $I_{1z}I_{2z}$ can therefore be selected while any other coherences will be discarded.

Selection of the coherence transfer pathway can be achieved using either a phase cycling routine or a pulsed field gradient route or a combination of both. Pulsed field gradients offer a superior method of coherence selection to phase cycling. Here the unwanted signals are dephased before reaching the receiver, solving problems associated with the dynamic range of the detector that are caused when dealing with large background signals. Furthermore signal selection can be achieved in a single scan.

The question of how to set up the gradients for coherence selection is answered using equation 2 where $\gamma_i$ is the gyromagnetic ratio, $p_i$ is the coherence number and $G_i\delta_i$ is the area of the gradient, $G_i$ being the gradient strength and $\delta_i$ the length of the gradient pulse. We note this approach can be applied to both homo and heteronuclear spins.

$$\sum \gamma_i p_i G_i \delta_i = 0 \quad (2)$$

If the zero quantum version of the experiment, 'OPSY-*z*', is to be selected, the coherence transfer pathway to be considered is $p_0 = 0 \rightarrow p_1 = 0 \rightarrow p_3 = -1$. Hence, a single gradient pulse after the first *RF* pulse will be sufficient to select zero quantum coherences as they are unaffected by the field gradients.

For the double quantum version, 'OPSY-d', two gradients or their equivalent that flank the second *RF* pulse are needed; the area of the second should be twice the area of the first. By using gradients with the same polarity an *N*-type experiment, $p_0 = 0 \rightarrow p_1 = +2 \rightarrow p_2 = -1$ is created. If the polarity of the gradients is reversed a *P*-Type experiment is performed where $p_0 = 0 \rightarrow p_1 = -2 \rightarrow p_2 = -1$. We have found experimentally that gradients of opposite polarity produce the better results. In addition, gradients of opposite polarity reduce eddy current generation.

This method can be bolted on in front of appropriate pulse sequences for use in both high resolution measurements and MRI based measurements.

If the starting point corresponds to a two spin singlet state, it is necessary to selectively excite one of the pairs of resonances in order to generate $I_z^A I_x^X$ and $I_x^A I_z^X$ magnetization, while leaving the $I_y^A I_y^X$ contribution unchanged. Also the terms $I_1 I_{2x}$ and $I_{1y} I_{2y}$ may be used as a starting point. If they are homo-nuclear this can be achieved through the application of two 90° pulses that are separated by a delay of $1/(4\delta v)$, where $\delta v$ is the separation between the two resonance frequencies. Here the relative phases of the two pulses are 135°, and the reference point is set to a point midway between the two resonances. If they are heteronuclear it is sufficient to apply a single 90° pulse to one of the spins. The result of this process is the creation of magnetisation of the type $I_z^A I_x^X$ and $I_x^A I_z^X$. Again suitable gradient and radio frequency selection schemes can be employed to differentiate these signals from those of the background and create them in the first place. It will be understood by the person skilled in the art that it is possible to use other pulse angles, whilst these may have lower affectivity, the measurements may be used and converted to observable magnetisation.

We note that in all of these approaches when higher order spin systems, such as the three spin system $I_{1z}I_{2z}I_{3z}$ are examined, similar approaches can again be used to select appropriate magnetization as a user experienced in the field would appreciate

One way to achieve OPSY-Slice selection is to use a series of 4 pulse combinations. Each of these uses the pulse scheme: radio-frequency pulse to transfer magnetisation into the transverse plane followed by the first coherence selection gradient followed by a second radiofrequency pulse followed by the second coherence selection gradient.

1) Both radio-frequency pulses are slice selective.
2) The first pulse is slice selective but the second is a 'broadband' pulse applied over a large frequency range and may or may not be in the absence of a magnetic field gradient.
3) The first radiofrequency pulse is a broadband pulse as described in (2) and the second is a slice selective pulse.
4) Both radiofrequency pulses are broadband pulses and the OPSY scheme is followed by a slice-selective pulse.

These can be used to replace the slice selective pulse in any magnetic resonance experiment that uses one. The substitution allows the magnetic resonance experiment to become sensitive only to those nuclei that possess the quantum coherence selected by the combination of the coherence selection gradients and other coherences will be dephased and will not be observed. This is applicable to, but not limited to, MRI experiments including Spin Echo and Gradient Echo based sequences including fast experiments based on Echo-planar Imaging (EPI), FISP (SSFP) and Turbo-spin-echo / Rapid acquisition with refocused echo sequences. Alternative sequences based on the separation of thermal and long lived magnetisation might also be envisaged which rely on this principle which employ rapid gradient cycling might also be achieved with optimal hardware. This is furthermore also applicable to localized spectroscopy experiments included but not limited to CSI (chemical shift imaging, spectroscopic imaging), PRESS, STEAM, ISIS and its derivates as well as for fast localized spectroscopy methods such as PEPSI and its derivates.

We further note that by using xyz gradients, applied at the magic angle, optimum suppression of the background is achieved since this also destroys intermolecular dipolar interactions (iMQC) which might otherwise survive the filtering method.

The method of the present invention utilises certain templates which bind or support an interaction with parahydrogen and a substrate. The templates may comprise a supported metal centre, a nanoscale structure or a material which are conventionally known by those skilled in the art. However, in a particular aspect of the present invention, the template is novel *per se.*

An example of such a template is provided by $[Ir(IMes)(H)_2(Py)_3]^+$; and analogues thereof which convery the same properties. In this case that is a metal centre which simultaneously binds $H_2$ and the material to be polarised. Exchange of these ligands facilitates substrate polarisation and can be controlled by varying both the ligand and the metal as a consequence of controlling the relative stability of the templates oxidation states.

Thus, according to a yet further aspect of the invention we also provide the templates comprising $[Ir(NHC)(H)_2(Py)_3]^+$, and analogues thereof, in which NHC is a saturated or unsaturated N-heterocyclic carbene.

We also provide the precursor to the template as hereinbefore described, which is $[Ir(COD)(IMes)(Py)]^+$.

In these examples, the precursor complex is stabilized by cyclooctadiene and pyridine. These ligands simply fulfil the role of ultimately providing access to $IrNHC^+$ which undergoes a net reaction to form $Ir(H)_2(NHC)(substrate)_2(L)^+$ or $Ir(H)_2(NHC)(substrate)_3^+$. The precursor might alternatively be stabilised by MeOD, acetone or acetone in conjunction with appropriate monoene or diene ligands. We further note that this method will also work with the previously filed example $Ir(H)_2(phosphine)(substrate)_3^+$ although the performance of the NHC is far superior.

Thus, according to the invention we provide a method wherein Py may be replaced in part or in full by the substrate that is to be the subject of the hyperpolarisation studies

The complex $[IrX(NHC)(COD)]$ may be prepared by reacting $[Ir(COD)OMe]$ with NHC.HX.

It will be understood by the person skilled in the art that IMes (1,3-dimesityl-imidazol-2-ylidene) is simply a representative example of an N-heterocyclic carbene. This ligand type acts as a strong sigma donor and therefore promotes the assembly of the template to support both $H_2$ and the substrate. We note also that the efficacy of this template can be controlled by changing the substituents on the carbene. For example the groups could be changed from 2,4,6 Me, to 2,6 iPr and to 2,6 Me. In this way specificity and efficacy might be addressed. We further note that the NHC backbone can be saturated in order to provide a further opportunity to impart control.

We further note that supported variants of these N-heterocyclic carbene complexes can be prepared, for example by using 1-methyl-3-(4-vinylbenzyl)imidazolium hexafluorophosphate and copolymerising it with styrene and divinylbenzene. When this material is then exposed to the iridium precursor, a polymer supported template is generated. In this way we ensure that the polarised materials and their polarisation template can be readily separated.

We note however that alternative precursors to it such as $[Ir(IMes)(COD)(MeOH)]BF_4$, or $[Ir(IMes)(COD)(Py)]PF_6$ (in which COD is cyclroocta-1,5-diene) or $[IrCl(IMes)(Py)_3]BF_4$ might also be employed. The important feature of the precursor to the template is simply that it acts as a source of $[Ir(NHC)]^+$, e.g. $[Ir(IMes)]^+$, which will then host the hydrogen and substrates necessary for polarisation transfer.

In another aspect of the invention we provide a templates comprising $[Ir(NHC)(H)_2(Py)_3]^+$; and analogues thereof, for use in PHIP magnetic resonance techniques as hereinbefore described.

In another aspect of the invention we provide a method of preparing a template $[Ir(COD)(NHC)(Py)_3]^+$, which comprises reacting a complex $[IrX(NHC)(COD)]$, in which X is an anion, with an excess of pyridine.

Typical transition metal atoms for the invention include Ru, Rh, Ir, W, Pd or Pt. Metal complexes, and in particular transition metal complexes, allow the attachment of numerous different symmetric molecules and compounds, in particular by coordinative bonding, and are therefore very important in practice.

Polarization transfer to the hyperpolarisable nuclei of the compound is easier to perform and can be applied to a broader scope of compounds in particular compounds that may not undergo a hydrogenation reaction.

Polarization may be transferred within the prepared fluid, which is enriched with symmetric molecules of a particular spin state (e.g. para-hydrogen enriched), directly to the hyperpolarisable nuclei of a compound, without altering the chemical identity of the compound in this process. To achieve this spin transfer, the invention proposes to use a template having sites of ordered environment, and the fluid and the compound are brought together in the presence of the template.

Such a site of ordered environment acts as a broker between a symmetric molecule (or its two halves) and a compound. A site of ordered environment first of all allows an arrangement of both a symmetric molecule and a compound at the site, i.e. it allows a bonding of some kind of a symmetric molecule and a compound to the site. Typically, the bonding is rather loose, and may be but is not limited to that of a coordinative type.

When a symmetric molecule is (or its two halves are) attached to a site of ordered environment, the two halves of the symmetric molecule become chemically or magnetically distinguishable; in other words the symmetry of the symmetric molecule is broken. This is a characteristic of the site of ordered environment according to the invention. In this situation, entropy will try to bring the nuclear spins of the two halves of the symmetric molecule closer to the thermal equilibrium; however the polarization of the symmetric molecule will need a destination to go to. As a consequence, the polarization of the symmetric molecule becomes - in principle - available for a transfer.

Further, when both a symmetric molecule (or its two halves) and a compound are arranged at a site of ordered environment, the site of ordered environment mediates (establishes) a coupling of the nuclear spins of the halves of the symmetric molecule and the compound (or its hyperpolarisable nuclei); this is another characteristic of the site of ordered environment according to the invention. The coupling mechanism may, in particular, be scalar coupling or dipolar coupling. By mediating the coupling, the compound becomes a possible destination of polarization to be transferred from the symmetric molecule. Typically, the site of ordered environment causes a close spatial neighbourhood of the spin-carrying atoms of the symmetric molecule and the hyperpolarisable nuclei of the compound on an atomic scale.

Basically by bringing together the prepared fluid, the provided compound and the template, and inducing an (at least) temporary association of the symmetric molecules, the compound and the template, the spin order transfer can be performed in a rather quick and simple way. In the simplest case, the fluid, the compound and the template can be mixed in solution, or a mixture of two components (such as the fluid and the compound) flows over or through the third component (typically the template). Under the conditions of the inventive method, the spin order transfer occurs in principle automatically, without a necessity for applying further measures. However, in accordance with the invention, the spin order transfer can be accelerated or intensified by some measures as described further below.

There is no net chemical reaction necessary between the symmetric molecules and the compound to be polarized in order to achieve the inventive spin order transfer, what makes the spin order transfer inherently simple. The chemical identity of the compound before and after the spin order transfer is the same, in accordance with the invention. For lack of the need for a chemical reaction, the inventive method becomes available for in principle every compound, in particular also compounds without double or triple C-C bonds necessary for conventional PHIP. Further, as compared to conventional PHIP, non-reactive symmetric molecules such as $(^{15}N)_2$ become available as a source of hyperpolarisation.

In accordance with the invention, the ordered environment may take the form of a homogeneous or heterogeneous polarization transfer catalyst. For example, the heterogeneous system may comprise a supported transition metal centre, a microscopic channel within a material such as a zeolite, a nanotube, or a suitable nanoparticle, a solvent with liquid crystalline properties, or any other feature that induces a short range magnetic differential with respect to the otherwise symmetric molecule and compound to be polarized.

In accordance with the invention, the compound is typically a molecule, but may be also an ion, a polymer, a nanoparticle, a supermolecular assembly, a peptide, a protein, or something else with a chemical identity. The chemical identity is defined by a chemical formula and a chemical (spatial) structure.

Note that TF - i.e. the temperature at which the symmetric molecules have their non-equilibrium spin distribution as defined in step a) - is typically the temperature at which the spin transfer of inventive step d) takes place. The non-equilibrium spin distribution of the symmetric molecules, which is still present when starting the spin order transfer of step d) in accordance with the invention, drives the spin order transfer.

Further below, examples are given for combinations of symmetric molecules, compounds and templates with sites of ordered environment. It should be mentioned that for a particular combination of symmetric molecules (as spin order source) and compound (as spin order destination, for the purpose of performing a magnetic resonance experiment on the hyperpolarized compound), a specific template must be chosen in order to accomplish the inventive method.

In one aspect of the invention the symmetric molecules may comprise para-hydrogen. Transferring spin order from para-hydrogen (p-$H_2$) to a compound in accordance with the invention is also referred to as "enhanced PHIP" here. Para-hydrogen is relatively inexpensive to prepare and can easily be arranged at different types of templates, and therefore is of great importance in practice. Typically p-$H_2$ is prepared as a fluid (liquid or gas) of p-$H_2$ enriched $H_2$. In alternative to p-$H_2$, the symmetric molecule can be a derivative of para-hydrogen with two hydrogen ligands whose nuclei are hyperpolarized. Further alternatives for symmetric molecules include e.g. $D_2$, $(^{15}N)_2$, oxalic acid, oxalate (HOOCCOOH), water and cis-1,2-diphenyl ethene.

In another aspect of the invention the sites of ordered environment each comprise a metal complex, in particular a transition metal complex. Typical transition metal atoms for the invention include Ru, Rh, Ir, W, Pd or Pt. Metal complexes, and in particular transition metal complexes, allow the attachment of numerous different symmetric molecules and compounds, in particular by coordinative bonding, and are therefore very important in practice.

In a further aspect of the inventive method, the template comprises a zeolite. Zeolites are in particular useful for a continuous preparation of hyperpolarized compound. For example, a zeolite, binding p-$H_2$, can be placed in a cell, with a flow of a solution of the compound over or through the zeolite. The suitability of zeolites for a spin order transfer from p-$H_2$ has been shown in experiment. Also note that zeolites may store fluid and/or compound in its cavities, which may be advantageous in the method of the present invention.

In a further aspect of the present invention, the hyperpolarisable nuclei of the compound include H, D, $^{29}Si$, $^{13}C$, $^{15}N$, $^{31}P$ and/or $^{19}F$. D indicates deuterium ($^2H$). Examples for spin order transfer in accordance to the invention with to above mentioned nuclei are detailed below. These nuclei are of particular importance in practice. Note that per compound (which is typically a molecule), there may be one hyperpolarisable nucleus or a plurality of hyperpolarisable nuclei.

In a further aspect of the invention the compound may be a metabolite. More generally, in accordance with the invention, the compound may be a substance to be found in or applicable to the human or animal body, in particular including drugs and prodrugs. This is particularly advantageous in medical applications.

The invention may be characterised in that the compound comprises an electron donor for attaching to a site of ordered environment, in particular wherein the electron donor is N, NH, S, P or O. The electron donor typically has one or more pairs of electrons, which can help establishing an interaction to a site of ordered environment, in particular by means of a coordinative bonding. Note that the compound comprises typically more atoms than those of the electron donor. For example, P may be hyperpolarized in phosphine, and C in $CO_2$, as shown in experiments.

In a further aspect the compound may be a gas, in particular ($^{13}C)O_2$. Inventive polarization transfer to $CO_2$ has been shown in experiment. Another compound in gaseous form may be ($^{15}N)_2$, in particular, for example, for inhalation by a patient.

In another aspect of the invention, at the end of step d), the compound comprising hyperpolarized nuclei may be separated from the site of ordered environment. This aspect may be particularly useful when the template would disturb or prohibit the magnetic resonance experiment on the compound, e.g. if the template degrades the magnetic resonance signal, or the template is toxic and the compound is to be inserted into a living human or animal body for an imaging experiment. If necessary, the separation can be enhanced by dedicated measures, in particular physical measures (e.g. dynamic pressure) or chemical measures (such as a pH alteration). It should be mentioned here that the spin order transfer, in accordance with the invention, can be established in a continuous flow experiment, such that at the same time some compound is in inventive step b), while some compound is in inventive step d), and some compound has already finished step d) and has been separated from the template.

In another aspect of the invention in step d) the spin order may be transferred spontaneously. In other words, the spin order is transferred from the symmetric molecules to the compound without applying an RF (=radio frequency) pulse sequence. This simplifies the spin order transfer enormously, since it can be performed outside of an NMR coil arrangement. Alternatively, an RF pulse sequence may be applied for supporting the spin order transfer from the symmetric molecule to the compound. In the latter case, in accordance with the invention, there is typically a low (i.e. non-zero, but for typical NMR experiments unsatisfactory) spontaneous spin order transfer which is increased by the application of the RF pulse sequence then. Note that the spin order transfer can be predominantly or completely induced (caused) by an RF pulse sequence, though (i.e. in the latter case without applying the RF pulse sequence, no polarization transfer would be observed). However, in accordance with the invention, it is highly preferred that at least some (and preferably all) of the spin order transfer is obtained without the application of an RF pulse sequence.

In another aspect of the invention, during step d), the entirety of the prepared fluid, the provided compound and the provided template brought together is agitated. Applying a fluid flow or fluid shear, for example, induced by shaking, in the entirety has been found to enhance spin order transfer; it is assumed that the exchange of symmetric molecules having transferred their spin polarization already, and/or the exchange of compound having received nuclear spin polarization, can be accelerated at the template in this way. Typical shaking of an NMR sample tube, which has led to an efficient spin order transfer in experiment, is a few seconds with a frequency of about 5 Hz, resulting in about 20 forth and back movements with an amplitude of several centimetres. Note that shaking can be done manually if desired or through $H_2$ bubbling through the solution.

Alternatively, a machine-induced oscillation or vibration or a sonic or ultrasonic r treatment may be applied. Further, bubbling a gas through a liquid may induce a beneficial fluid flow or shear in accordance with the invention.

In a further development of the above aspect, during shaking the entirety is exposed to a magnetic field, preferably wherein the field strength is 1 T or less, most preferably wherein the field strength is between 0T and 1 T.

Experiments have shown that a relative movement of the entirety and a magnetic field can enhance the spin order transfer. Such a relative movement can be established by shaking the entirety in a magnetic field. For shaking, the magnetic field is typically static, and earth magnetic field is enough for good transfer efficiency. The magnetic field in this further development may be inhomogeneous in accordance with the invention. In accordance with the invention, the magnetic field conditions during step d) can be used to influence or even control the spin order transfer, in particular the phase of the final, enhanced NMR-signal.

Moreover, in a further aspect of the invention, during step d), the entirety of the prepared fluid, the provided compound and the provided template brought together is exposed to a magnetic field. The magnetic field may optionally comprise a changing magnetic field, such as an oscillating magnetic field, in particular wherein the amplitude of the field strength of the oscillating magnetic field is between 20 $\mu$T and 0.1 T. By altering the magnetic field, a relative movement of the entirety and the magnetic field can be established with a stationery entirety. In experiment, manually moving a hand-held permanent magnet several times back and forth near an NMR tube (i.e. near the entirety) has resulted in good spin order transfer. The oscillating magnetic field may be inhomogeneous across the entirety (NMR sample tube), in accordance with the invention. Note that a typical oscillation frequency in accordance with the invention is about 1 to 10 Hz but can lie outside this range when necessary. We further note the change in field can follow a smooth variation or a series of steps to achieve optimal polarisation transfer or may employ a non-zero DC field of between 20 $\mu$T and 0.1 T.

Further, the chemical identity of the compound as prepared in step b) is the same as the chemical identity of the compound as subject to the NMR measurement of step e). This aspect is particularly simple. Alternatively, in accordance with the invention, between steps d) and e) the compound may undergo a chemical reaction; however this chemical reaction is independent of the spin order transfer of step d).

Also within the scope of the present invention is the use of the method or one of its variants in a magnetic resonance imaging experiment, in particular wherein the compound is used as a contrast agent. The hyperpolarized nuclei of the compound may be used for image formation. The invention can, in particular, be used to obtain images of a living human or animal body or parts of it, in order to prepare medical diagnostics or therapy. Typically, the compound is applied to the human or animal body after having undergone inventive step d).

According to the invention, by suitably arranging parahydrogen or a derivative thereof and a hyperpolarisable nucleus or nuclei in an ordered environment, hyperpolarisation can be transferred directly from the parahydrogen nuclei to the hyperpolarisable nucleus, i.e. without the need to chemically incorporate the parahydrogen into a compound comprising the hyperpolarisable nucleus. The hyperpolarized state of the nucleus/nuclei is substantially retained when the compound is removed from the ordered environment.

Also provided is a hydrogenated metal complex comprising a pair of hydride ligands whose nuclei are hyperpolarized and a ligand comprising a hyperpolarisable nucleus, wherein the hydride ligands are arranged such hyperpolarisation is directly transferable to the hyperpolarisable nucleus.

The compounds according to this aspect of the invention may be useful as magnetic resonance (MR) imaging agents. Accordingly, the use of compounds polarized in this way in diagnosis or therapy also forms part of the invention. In one aspect, the invention provides a composition comprising a compound comprising a nucleus which has been hyperpolarized by a process of the invention and a physiologically acceptable carrier or excipient.

According to the present invention, parahydrogen or a hyperpolarized derivative thereof and a compound comprising a hyperpolarisable nucleus are arranged in an ordered environment such that hyperpolarisation is directly transferable from the parahydrogen or derivative to the hyperpolarisable nucleus. The hyperpolarisable nucleus is then hyperpolarized by directly transferring hyperpolarisation from the parahydrogen or derivative to the hyperpolarisable nucleus. The compound comprising the hyperpolarized nucleus can then be removed from the ordered environment and used as required, e.g. as a contrast agent.

A process of the invention utilises parahydrogen or a hyperpolarized derivative thereof. The hyperpolarized derivative may comprise a pair of hydride ligands whose nuclei are hyperpolarized. Hyperpolarisation is transferred directly from the parahydrogen or derivative to a compound comprising one or more hyperpolarisable nuclei. By way of example, the or each hyperpolarisable nucleus may be selected from $^{1}$H, $^{13}$C, $^{15}$N, $^{29}$Si and $^{31}$P nuclei. An ordered environment is utilised to ensure that the parahydrogen or derivative and the hyperpolarisable nucleus are suitably arranged so as to facilitate direct transfer of hyperpolarisation.

The compound comprising the hyperpolarisable nucleus may be organic or inorganic in nature. Typically, the compound will comprise one or more atoms selected from hydrogen, carbon, nitrogen, oxygen, silicon, sulphur, fluorine and phosphorus. Where the compound is a ligand, it may be a mono-, bi- or multi-dentate ligand. Included are compounds, especially ligands, comprising one or more heterocyclic groups, in particular one or more heterocyclic groups comprising $^{15}$N. For example, the compound may comprise one or more pyridine groups [Ir(NHC)(H)$_2$(Py)$_3$]$^{+}$.

Hydrogenation of the complex may be achieved by contacting the complex with a fluid, typically a solution, containing dissolved para-hydrogen, preferably such that the resulting hydride ligands are in equilibrium with the para-hydrogen in solution. Fluids enriched with para-hydrogen are particularly suitable in this regard. The term "enriched hydrogen" as used herein includes reference to hydrogen in which there is a higher than equilibrium proportion of para-hydrogen, for example where the proportion of para-hydrogen is more than 25%, e.g. more than 30%, e.g. 45% or more, e.g. 60% or more, e.g. 90% or more, in particular 99% or more. Enriched hydrogen may be obtained catalytically at low temperatures e.g. at 160 K or less, preferably at 80 K or less or more preferably at about 20 K. The parahydrogen thus formed may be stored for long periods, preferably at low temperature, e.g. 18 to 20 K. Alternatively the parahydrogen may be stored in pressurized gas form in containers with nonmagnetic and non-paramagnetic inner surfaces, e.g. a gold or deuterated polymer coated container. Parahydrogen may also be obtained by electrolysis. The hydrogenation step may be performed in the liquid or gaseous phase, and preferably in the absence of materials which would promote relaxation.

The ligands are arranged such that the hyperpolarisation is directly transferable from the hydride ligands to hyperpolarisable nucleus, i.e. hyperpolarisation is transferable without first chemically incorporating the hydride ligands into the compound comprising the hyperpolarisable nucleus. When a metal complex is hydrogenated with para-hydrogen, the resulting hydride ligands are normally formed in a *cis* arrangement. In this arrangement, transfer of hyperpolarisation from the hydride ligands to the hyperpolarisable nucleus will normally be possible, especially when the ligand comprising the hyperpolarisable nucleus is located *trans* to the hydride ligands.

Hyperpolarisation of the hyperpolarisable nucleus by the parahydrogen or derivative ligands may occur spontaneously. In general, spontaneous polarization will occur when the transitions associated with the NMR signals are close in energy and therefore mix. This situation can be readily achieved in a low field, but may also be achieved by the application of a suitable train of radio frequencies.

Spontaneous transfer can in some cases be enhanced further by pulse sequences of electromagnetic radiation that can be applied to the system which will result in polarization transfer. Examples of suitable sequences can be found in the Figures herein and in Blazina et al, Dalton Trans., 2004,2601-2609.

After hyperpolarisation has been transferred, the compound comprising the hyperpolarized nucleus can then be separated from the ordered environment and the parahydrogen or derivative thereof. Separation may be achieved using physical and or chemical means. Where a hydrogenated metal complex forms the ordered environment, the ligand comprising the hyperpolarized nucleus is separated from the complex. In this regard, the ligand is preferably chemically or physically labile. Where the ligand is labile, dissociation of the ligand from the complex may be achieved by contacting the complex with a solution comprising the ligand in unbound form. Equilibrium may be established between the bound and unbound ligand, facilitating dissociation of the hyperpolarized ligand from the nucleus.

Hyperpolarized compounds of the invention may be suitable for use in high resolution NMR experiments. In this case, the compound should preferably be strongly polarisable (for example, to a level of greater than 5%, preferably greater than 10%, more preferably greater than 25%). Collection of $^1H$, $^{13}C$, $^{15}N$, $^{31}P$ or $^{29}Si$ signals should be facilitated.

The invention is particularly suited to the production of MR imaging agents. In this case, the compound should preferably be strongly polarisable (for example, to a level of greater than 5%, preferably greater than 10%, more preferably greater than 25%) and have a non-hydrogen MR imaging nucleus with a long T1 relaxation time under physiological conditions, e.g. $^{13}C$, $^{15}N$ or $^{29}Si$. By a long $T_1$ relaxation time is meant that $T_1$ is such that once polarised, the MR imaging agent will remain so for a period sufficiently long to allow the imaging procedure to be carried out in a comfortable time span. Significant polarization should therefore be retained for at least 1 s, preferably for at least 60 s, more preferably for at least 100 s and especially for 1000 s or longer. Furthermore, the chemical shift, or even better the coupling constant of the signal from the imaging nucleus should preferably be influenced by physiological parameters (e.g. morphology, pH, metabolism, temperature, oxygen tension or calcium concentration or other physical phenomenon such as ligand binding or hydrogen bonding). For example, influence by pH can be used as a metabolic marker, whilst changes in oxygen tension may be a disease marker such as a cancer marker. Alternatively, the MR imaging agent may conveniently be a material which is transformed (e.g. at a rate such that its half-life is no more than 10 $xT_1$ of the reporter nucleus, preferably no more than 1 $xT_1$) in the subject under study to a material in which the reporter nucleus has a different coupling constant or chemical shift.

The MR imaging agents may be administered to a sample and the sample subsequently exposed to radiation of a frequency selected to excite nuclear spin transitions of one or more hyperpolarized nuclei present in the imaging agent. The magnetic resonance signals of the nuclei can then be detected. The detected signals can then be used to generate an image, biological functional data or dynamic flow data.

MR imaging agents may be used to image a subject, for example, selected from a human or animal, a cell culture, a membrane-free culture or a chemical reaction medium. Thus, it may be preferable for the MR imaging agents to have negligible toxicity. Such agents have both *in vitro* and *in vivo* usage.

The MR imaging agent may be administered parenterally, e.g. by bolus injection, by intravenous or intra-arterial injection or, where the lungs are to be imaged, in gas or spray form, e.g. by aerosol spray. Oral and rectal administration may also be used.

MR imaging agents may be conveniently formulated with conventional pharmaceutical or veterinary carriers or excipients. Formulations of the invention may thus comprise one or more components selected from stabilizers, antioxidants, osmolality adjusting agents, solubilising agents, emulsifiers, viscosity enhancers and buffers. Preferably, these components are not paramagnetic, superparamagnetic, ferromagnetic or ferrimagnetic. The formulation may be in forms suitable for parenteral (e.g. intravenous or intra-arterial) or enteral (e.g. oral or rectal) application, for example for application directly into body cavities having external voidance ducts (such as the lungs, the gastrointestinal tract, the bladder and the uterus), or for injection or infusion into the cardiovascular system. However, solutions, suspensions and dispersions in physiological tolerable carriers (e.g. water) will generally be preferred.

Where the MR imaging agent is to be injected, it may be convenient to inject simultaneously at a series of administration sites such that a greater proportion of the vascular tree may be visualized before the polarisation is lost through relaxation. Intra-arterial injection is useful for preparing angiograms and intravenous injection for imaging larger arteries and the vascular tree.

Parenterally administrable forms should of course be sterile and free from physiologically unacceptable agents and from paramagnetic, superparamagnetic, ferromagnetic or ferrimagnetic contaminants, and should have low osmolality to minimize irritation or other adverse effects upon administration and thus the formulation should preferably be isotonic or slightly hypertonic. Suitable vehicles include aqueous vehicles customarily used for administering parenteral solutions such as sodium chloride solution, Ringer's solution, dextrose solution, dextrose and sodium Chloride solution, lactated Ringer's solution and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975). The compositions can contain preservatives, antimicrobial agents, buffers and antioxidants conventionally used for parenteral solutions, excipients and other additives which are compatible with the MR imaging agents and which will not interfere with the manufacture, storage or use of the products.

For use in *in vivo* imaging, the formulation, which normally will be substantially isotonic, may conveniently be administered at a concentration sufficient to yield a 1 $\mu$M to 1M concentration of the MR imaging agent in the imaging zone. However, the precise concentration and dosage will of course depend upon a range of factors such as toxicity, the organ targeting ability of the MR imaging agent, and the administration route. The optimum concentration for the MR imaging agent represents a balance between various factors. In general, optimum concentrations will typically range from about 0.1 mM to about 10 M, especially from about 0.2 mM to about 1 M, more especially from about 0.5 mM to about 500 mM. Formulations for intravenous or intra-arterial administration may, for example, contain the MR imaging agent in concentrations of from about 10 mM to about 10 M, especially from about 50 mM to about 500 mM. For bolus injection the concentration may conveniently range from about 0.1 mM to about 10M, especially from about 0.2 mM to about 10 M, in particular from about 0.5 mM to 1 M, more particularly from about 10 mM to about 500 mM, yet still more particularly from about 10 mM to about 300 mM.

The dosages of the MR imaging agent used according to the process of the present invention will vary according to the precise nature of the MR imaging agents used, of the tissue or organ of interest and of the measuring apparatus. Typically, the dosage should be kept as low as possible whilst still achieving a detectable contrast effect. By way of example, the dosage may range from 1 to 1000 mg/kg, e.g. from 2 to 500 mg/kg, especially from 3 to 300 mg/kg.

Once the MR imaging agent has been administered to the subject, the MR signals may be detected using procedures known in the art. For example, it may be advantageous to use fast single shot imaging sequences e. g. EPI, RARE, FSE or turboFLASH. MR signals may be conveniently converted into two- or three-dimensional image data or into functional, flow or perfusion data by conventional manipulations. By imaging, it will be appreciated that not just production of two- or three-dimensional morphological images is covered. The images produced may be representations of the value or temporal change in value of a physiological parameter such as temperature, pH, oxygen tension and the like. Morphological images however will generally be produced. For *in vivo* imaging, the MR imaging agent should of course be physiologically tolerable or be capable of being presented in a physiologically tolerable form.

The process hereinbefore described can provide that the reaction is conducted in a fluid containing parahydrogen.

It can be provided that the fluid contains parahydrogen-enriched hydrogen.

Further, it can be provided that the hydride ligands in the template are in equilibrium with the hydrogen in said solution.

Further, the process can provide that the hydride ligands are in a *cis* arrangement.

The process can provide that the ligand is attached to the metal *via* an atom containing said hyperpolarisable nucleus.

The process can also provide that the metal complex is present in a solution comprising said ligand in unbound form.

In the latter case, the process can provide that the bound ligand is labile and in equilibrium with unbound ligand.

The process can provide that the complex is bound to a support.

Further, the process can provide that the complex is a transition metal complex.

In the latter case, it can be provided that the complex comprises a transition metal selected from Ru, Rh, Ir, W, Pd and Pt.

Further, the process can provide that process the compound comprising the hyperpolarisable nucleus is a metabolite, a drug or a prodrug.

Further, the process can provide that the hyperpolarisable nucleus is a $^1$H, $^{29}$Si, $^{13}$C, $^{15}$N or $^{31}$P nucleus.

The process can provide that the hyperpolarisation is transferred spontaneously.

The process can also provide that the hyperpolarisation is transferred using a pulse sequence.

The device may provide that the metal complex is hydrogenated with parahydrogen.

The device may be further comprising an inlet for a solution comprising a ligand in unbound form, wherein the ligand is a compound comprising a hyperpolarisable nucleus.

The device may be further comprising an outlet for one or more fluids. A compound as mentioned above is useful as a template to produce a polarised material for use as magnetic resonance (MR) contrast agent. A compound as mentioned above may be combined with physiologically acceptable carrier or excipient.

We note that we used the terms singlet state or long lived here to refer to a coupled pair of spins, I and S, in the state IzSz+IxSx+IySy. This state is formed through magnetisation transfer from parahydrogen, a true singlet state due to the rotation and nuclear wavefunction description that's results from symmetry restrictions of the total wavefunction for a fermion.

We note that the magnetisation generated will also include a varying amount of longitudinal spin order terms of the type IzSz.

The generation and observation of these two types of magnetisation, and their higher spin order, highly correlated longitudinal magnetisation counterparts such as IzSzRz are the subject of this patent.

The oxidative addition of dihydrogen to a metal centre is a common reaction in inorganic chemistry and is a key step in many hydrogenation reactions. In the field of parahydrogen, the reactions of 16-electron complexes such as $IrCl(CO)(PPh_3)_2$ with $H_2$ have been extensively examined. This process follows a concerted pathway, with addition over the OC-Ir-Cl axis placing the hydrides in a mutually cis orientation, but the increased detection limits allow polarized hydride resonances to be observed for both the *cis-trans*- $IrH_2(CO)(PPh_3)_2Cl$ isomer and a previously undetected *ciscis*-$IrH_2(CO)(PPh_3)_2Cl$ product. This in turn tells us that $H_2$ addition over the P-Ir-P axis of $IrCl(CO)(PPh_3)_2$ is possible. The successful observation of this new species using the *para*-$H_2$ approach provides one illustration of the many that are now reported, which show that it is possible to see previously unobserved complexes via this route. Trihydrides such as $IrH_3(eO)_xCPR_3)_y$ (x =2 and y =1;x =1 and y =2; $PR_3$ =$PPh_3$, $PMe_3$ and $AsMe_2Ph$) can be examined with *para*-$H_2$.

Signals due to $Ir(H)_2(PPh_3)_2(Py)$, have also been detected with *para*-$H_2$ enhancement. The detection of hydride resonances of $Ir(H)_2(PPh_3)_2(Py)$, when the pyridine concentration is 0.1 $\mu M$ corresponds to the detection of a signal that is diagnostic of 50 pmol of substrate and illustrates the potential power of this sensing method in detected analytes such as benzimidazole, purine, and adenine.

UV photolysis of samples within an NMR probe can be used to generate materials that can subsequently be characterized using conventional NMR methods at low temperatures, while others have used photo-CIDNP to determine kinetic parameters. The use of in situ irradiation methods allowed the monitoring of chemical reaction between $Ru(CO)_3(L_2)$ (where $L_2$ = dppe=1,2-(bis)diphenylphosphino ethane or $L_2$ =dpae=1,2-bis(diphenylarsino)ethane) and pure parahydrogen (generated at 18 K) in a very precise way and the quantification of the scale of the parahydrogen effect *in situ* UV irradiation of solutions containing $Ru(CO)_3(L)_2$, where L=$PPh_3$, $PMe_3$, $PCy_3$ and P(p-tolyl)$_3$ and *para*-$H_2$, involves two reactions, the loss of CO and the formation of the *cis-cis-trans*-L isomer of $Ru(CO)_2(L)_2(H)_2$; and the formation of *cis-cis-cis* $Ru(CO)_2(L)_2(H)_2$ and $Ru(CO)_2(L)(solvent)(H)_2$ where solvent=toluene, tetrahydrofuran (THF), and pyridine. In the case of L=$PPh_3$, the normally invisible species *cis-cis-trans*-L $Ru(CO)_2(L)_2(H)_2$ can be shown to be an effective hydrogenation catalyst, with rate limiting phosphine dissociation proceeding at a rate of 2.2 s-1 in pyridine at 355 K. The use of *in situ* photochemistry in conjunction with *para*-$H_2$, therefore, provides a further opportunity to detect the otherwise invisible species.

A particularly important class of reactions studied by *para*-$H_2$ corresponds to metal-catalyzed hydrogenation. Hydrogenation can, therefore, proceed via the pairwise transfer of H2 to the alkene. By demonstrating that $RuHCl(PPh_3)_2$ could not itself be the active catalytic intermediate, it has been proposed that the formation of $RuH_2(PPh_3)_2$ is possible. Thus *para*-$H_2$-enhanced NMR demonstrates that one of the pathways of $RuHCl(PPh_3)_3$-catalyzed hydrogenation involves a small but active quantity of $RuH_2(PPh_3)_2$. Studies on the hydrogenation catalyst $[RhCl(PPh_3)_2]_2$ have revealed that the binuclear dihydride $Rh(H)_2(PPh_3)_2(\mu$-Cl$)_2Rh(PPh_3)_2$ and the tetrahydride complex $[Rh(H)_2(PPh_3)_2(\mu$-Cl$)]_2$ are readily formed upon reaction with *para*-$H_2$. When these reactions are examined in the presence of an alkene and parahydrogen, signals corresponding to binuclear complexes of the type $Rh(H)_2(PPh_3)_2(\mu$-Cl$)_2(Rh)(PPh_3)$(alkene) can be detected. Magnetisation transfer from the hydride ligands of these complexes into the alkyl group of the hydrogenation product can be observed and it has been proposed that hydrogenation was proposed proceeds via binuclear complex fragmentation and trapping of the resultant intermediate $RhCl(H)_2(PPh_3)_2$ by the alkene. When the analogous iodide complexes $[RhI(PPh_3)_2]_2$ and $RhI(PPh_3)_3$ are examined, $Rh(H)_2(PPh_3)_2(\mu$-I$)_2Rh(PPh_3)_2$, $[Rh(H)_2(PPh_3)_2(\mu$-I$)]_2$ and $Rh(H)_2I(PPh_3)_3$ are observed in addition to the corresponding binuclear alkene dihydride products. Such studies therefore clearly demonstrate the potential of the *para*-$H_2$ approach to offer new insights into hydrogenation reactions.

The parahydrogen effect can be used to enhance the NMR signals of ligands other than the hydrides that are directly attached to a metal centre and therefore metal complexes can be detected through the sensitisation of the organic ligands framework.

The optimization of magnetization transfer pathways has become an important aspect of research in the field of magnetic resonance imaging because of the use of such hyperpolarized materials in $^{13}$C MRI studies. Examples of these have included the use of polarized hydroxyethylpropionate, where the T1 of the carbonyl group is 138 s and 11% net polarization was achieved, to monitor the pulmonary vasculature and pulmonary perfusion through true FISP measurements. The steady-state free precession (SSFP) technique has been used with the same substrate, to demonstrate that coronary angiography data can be collected with similar success using this approach. Imaging studies of succinate,

using a 3D $^{13}$C fast imaging employing steady-state acquisition (FIESTA) sequence, have also been achieved, including some within the brain of normal and tumour-bearing rats. Parahydrogen-based signal amplifications have also been used to study propane gas that is generated through a heterogeneous reaction. The potential of this approach for use in the imaging of lungs and other porous materials, and of the catalysis process itself, will clearly be explored in the future.

The invention will now be illustrated by way of example only and with reference to the accompanying drawings, in which Figure lis a scan of NMR spectra for the template Ir(Mes)(H)2(nicotinamide)$_3$$^+$;
Figure 2 is an illustration of Unlocked $^1$H NMR spectrum recorded in protio-CH$_2$Cl$_2$ using the OPSY filtration sequence to suppress background signals;
Figure 3 is a single shot True-FISP $^1$H MRI images of an 8 mm sample tube containing cylindrical rods with 1 mm internal diameter;
Figure 4 is a single shot OPSY filtered True-FISP $^1$H MRI images of an 8 mm sample tube containing cylindrical rods with 1 mm internal diameter;
Figure 5 is a schematic of the building block of the pulse sequence used; and
Figure 6 is a schematic of the pulse sequence in high resolution measurements.

sScheme 1 is a schematic representation of the reaction of 1 with pyridine and H2.

sFig. 1 is the hydride region of 1H NMR spectra.

sFig. 2 illustrates selected regions of polarized 1H NMR spectra.

sFig. 3 illustrates selected regions of polarized 1H NMR spectra.

sFig. 4 is a plot of the amplitude versus the polarising field strength.

sFig. 5 is a plot of the amplitude versus the polarising field strength.

sFig. 6 1H True FISP MRI images of an 8 mm sample tube at 600 MHz.

sFig. 7 1H True FISP MRI images of an 8 mm sample tube at 600 MHz.

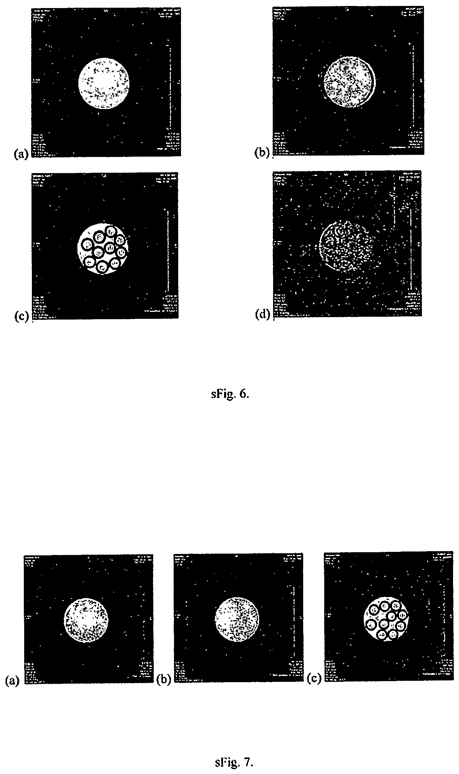

Fig. 7 is a plot of relative amplitude versus mixing field.

Fig. 8 is a plot of relative amplitude versus mixing field.

Fig. 9 is a plot of relative amplitude versus mixing field.

Fig. 10 is a plot of relative amplitude versus mixing field.

Fig. 11 is a plot of relative amplitude versus mixing field.

Figure legends:

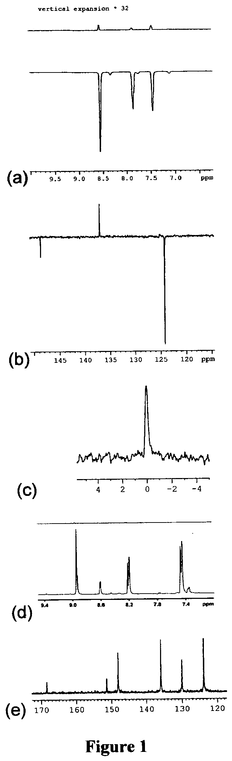

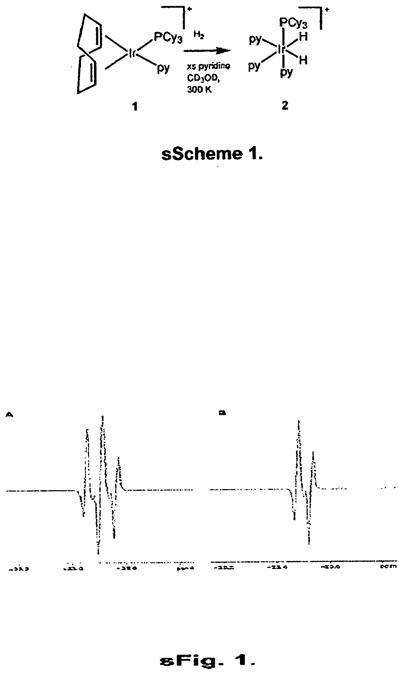

Figure **1.** One scan NMR spectra of a sample containing a templating medium, 6 nano moles of pyridine and *para*hydrogen at 295 K in d$_4$-methanol: a) (top) Single scan $^1$H control trace with vertical expansion 32 higher than the (lower) $^1$H trace which was taken immediately after shaking in a 200 gauss field; and (b) $^1$H decoupled $^{13}$C, single scan trace with refocusing obtained immediately after shaking in a 200 gauss field; (c) $^{15}$N, $^1$H decoupled trace with refocusing using 25 nano moles of $^{15}$N labelled pyridine after shaking in a 200 gauss field; (d) $^1$H NMR trace of 50 $\mu$moles of nicotinamide polarised in this way; (e) $^{13}$C, $^1$H decoupled single scan trace with refocusing in magnitude obtained immediately after shaking.
As a consequence when the substrate, nicotinamide, is probed by NMR spectroscopy, enhanced NMR signals result in the $^1$H, $^{13}$C and $^{15}$N domains.

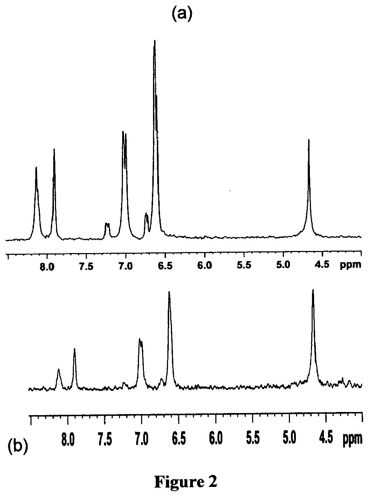

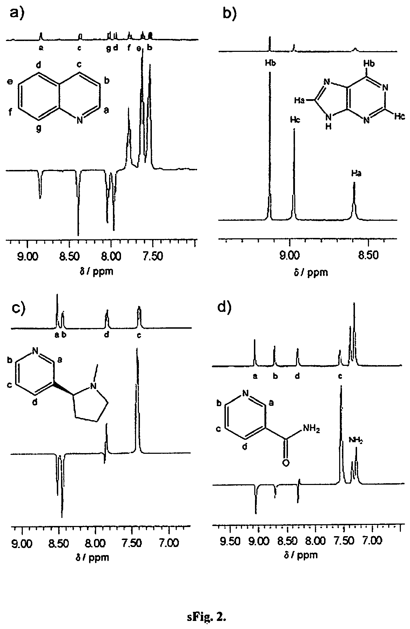

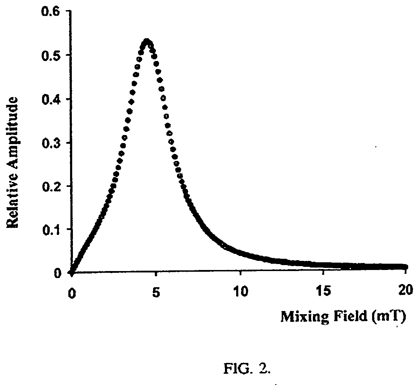

2) These signals possess NMR characteristics that make them suitable for examination by MRI and NMR spectroscopy using novel pulse sequences because the magnetic states that are produced differ from those routinely achieved when thermal
magnetisation is created due to the interaction with the magnetic field of the instrument that is used for observation.
3) More specifically, it is possible to create a singlet state for all pairs of coupled spins. This singlet state is characterised by a long lifetime in low magnetic field and exemplified by parahydrogen itself. In addition to the long lifetime of this state, its unique NMR properties mean that it can be interrogated while removing contributions from the other thermal magnetisation that is present in the sample; for example water, lipid or fat signals. In a magnetic field the singlet state will evolve under chemical shift into a form of longitudinal spin order. These results shown here correspond to the interrogation of these states but we note that the singlet state itself can also be interrogated and deliver similar results.
We illustrate that our spontaneous polarisation approach generates such states in the spectra shown in Fig 2. These are recorded in a protio solvent, in this case methylene chloride, and the polarised signals correspond to those of free polarised pyridine. The two traces differ simply in their point of observation. In the first, the polarised magnetisation is selected using the appropriate pulse sequence and read immediately after the polarisation step. In the second, we wait 150 seconds before introducing the sample and making the measurement. In the second trace, signals for the three proton sites or pyridine are still visible even though the polarisation transfer step occurred 150 seconds before observation. These results therefore demonstrate experimentally that the interaction of parahydrogen with the substrate while supported on the host results in the creation of long lived NMR states. Furthermore they demonstrate that they can be observed even in protio media when an appropriate pulse sequence is employed to remove the thermal magnetisation.

Figure **2.** (a) Unlocked $^1$H NMR spectrum recorded in protio-$CH_2Cl_2$ using the OPSY filtration sequence to suppress background signals and thereby enable the detection of 60 $\mu$moles of polarised pyridine in a single scan; (b) $^1$H NMR spectrum recorded in neat protio-$CH_2Cl_2$ using the OPSY filtration sequence to suppress background signals and thereby enable the detection of 60 $\mu$moles of polarised pyridine in a single scan that was recorded 150 seconds after the completion of the polarisation step in order to demonstrate the creation of long lived spin states.

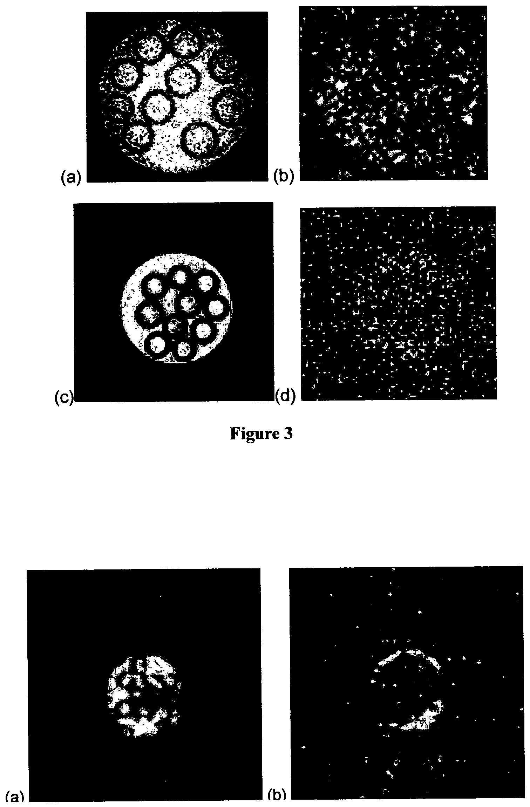

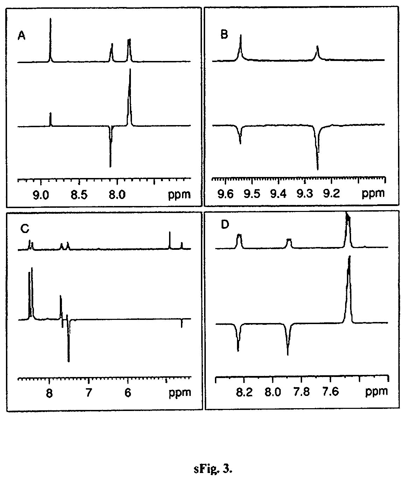

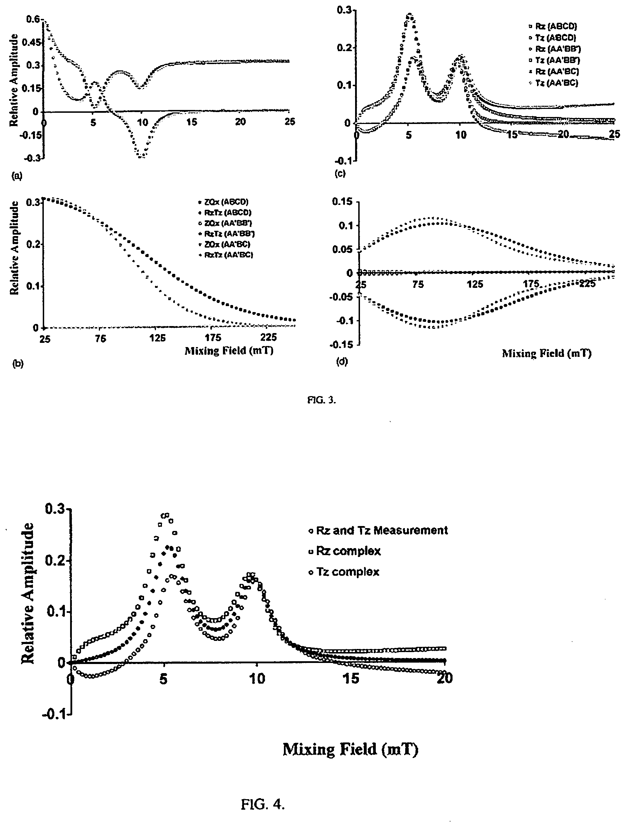

4) In this way, a naturally occurring contrast is created for the polarised product resonances (cf. nicotinamide) that can be readout at the expense of the signals normally described as those corresponding to the thermal background. This result ensures that an optimised image or spectrum can be produced when the system is examined with an appropriately modified pulse sequence. The term pulse sequence here simply describes these series of steps that are under taken to encode the magnetisation in a form that provides the observable signal that is then subject to analysis. Figure 3 illustrate that MRI based images can be collected for this magnetisation in methanol-$d_4$.

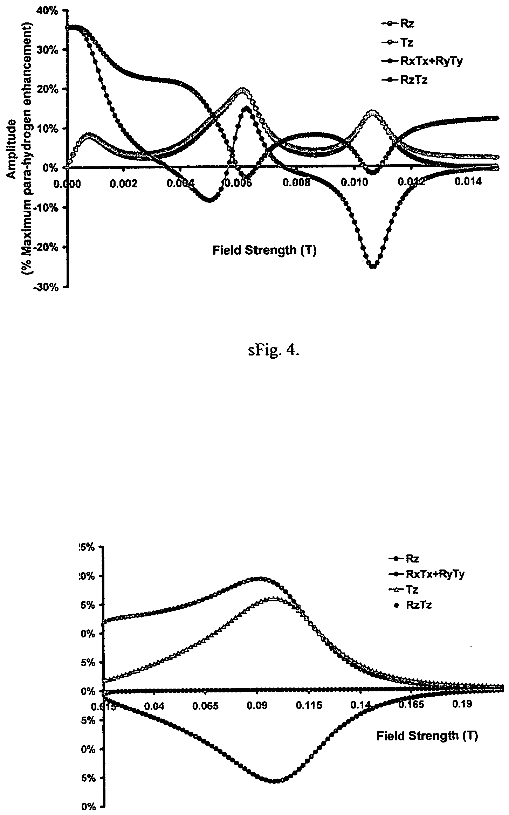

Figure **3.** Single shot True-FISP $^1$H MRI images of an 8 mm sample tube containing cylindrical rods with 1 mm internal diameter showing (a) polarised pyridine derived $^1$H NMR signal in $d_4$-methanol at 400 MHz within a 0.5 mm slice as compared to (b) a 20 mm slice using the unpolarised material. (c) polarised nicotinamide derived $^1$H NMR signal in $d_4$-methanol at 600 MHz within a 5 mm slice as compared to (d) a 5 mm slice using the unpolarised material. We illustrate this by showing a more traditional MRI trace, corresponding to the slice of a TRUE FISP map in figure 4. The spectra were collected for pyridine, and nicotinamide and are illustrated in both methanol$_{d4}$ and protio methylene dichloride solvent systems. The detected signals therefore correspond to the polarised resonances for the two substrates.

Figure **4**. Single shot OPSY filtered EPI $^1$H MRI images of an 8 mm sample tube containing cylindrical rods with 1 mm internal diameter showing (a) polarised nicotinamide derived $^1$H NMR signal in a doped protio methanol sample at 600 MHz within a 6 mm slice as compared to (b) a 6 mm slice using the unpolarised material.

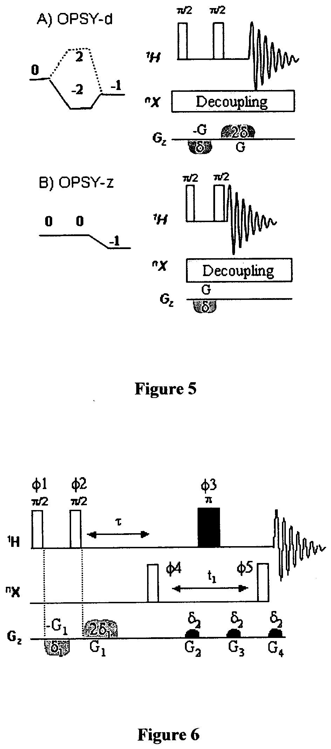

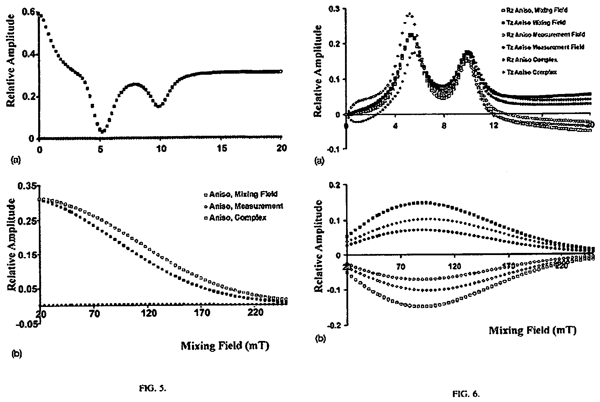

Figure **5.** Double quantum OPSY (OPSY-d). Phase cycle: first pulse : $(y)_4$ $(x)_4$ second pulse: $(x)_4$ $(y)_4$, receiver: : $(x)_4$ $(y)_4$. b) Zero quantum OPSY (OPSY-z). Phase cycle: first pulse: x second pulse: x, receiver: x. Gradient strength (G): 57 G cm$^{-1}$, first gradient duration for both experiments ($\delta$) 1 ms, second gradient duration ($2\delta$) for the double quantum version, 2 ms. Sine-shaped gradients were used. 0.5 ms stabilization delays were used after each gradient. Decoupling is optional. The usual inter-scan delay intended to let the sample relax is of no use in OPSY sequences since it decreases the amount of signal recordable per unit of time, see text for details. On the left: representation of the coherence transfer pathway. Dotted lines represent discarded pathways.

Figure **6.** The double quantum filtered HMBC experiment (OPSY-*d*-HMBC). If a zero quantum filtration is desired, the second gradient pulse must be omitted. The usual inter-pulse recycle delay is unnecessary since the hyperpolarisation effect is lost through relaxation. Phase cycle: $\phi_1$ = x, $\phi_2$ = x, $\phi_3$ = x x -x -x, $\phi_4$ = x -x, $\phi_5$ = $(x)_4$ $(-x)_4$, $\phi_{rec}$ = (x -x)$_2$ (-x x)$_2$. Sine-shaped gradients were used. G$_1$ = 57 G.cm$^{-1}$. $\delta_1$=$\delta_2$= 1 ms. G$_2$: G$_3$: G$_4$ 50:30:40.1 for $^{13}$C selection, 70:30:50.1 for $^{15}$N selection and 30:30:24.3 for $^{31}$P selection. Hollow bars and filled bars represent $\pi$/2 and $\pi$ pulses respectively. t = 1/(2J$_{CH}$).

**sScheme 1.** Reaction of 1 with pyridine and H2 in d4-methanol leads to 2.

**sFig. 1.** Hydride region of 1H NMR spectra collected during the reaction of 1 with 3atm. para-H2 in d4-methanol at 295 K in the presence of 15N-pyridine without (a) and with (b) 31P decoupling.

**sFig. 2.** Selected regions of polarized 1H NMR spectra of the indicated substrates with resonances as attributed: a) quinoline; (b) purine; (c) nicotine; (d) nicotinamide as polarized using the PCy3 template.

**sFig. 3.** Selected regions of polarized 1H NMR spectra of the indicated substrates: (a) quinoxaline; (b) quinazoline; (c) 3-fluoro-pyridine (the upper trace has a vertical expansion of x 16 relative to the lower trace); (d) dibenzothiophene

**sFig. 4.** Product operator terms created by magnetisation transfer within an AA'BC spin system from para-H2 (AA') to the substrate RT (BC), where both R and T are hydrogen atoms, as a function of the polarising field strength over the range 0 to 0.015 T.

**sFig. 5.** Product operator terms created by magnetisation transfer within an AA'BC spin system from para-H2 (AA') to the substrate RT (BC), where both R and T are hydrogen atoms, as a function of the polarising field strength over the range 0.015 to 0.2 T.

**sFig. 6.** 1H True FISP MRI images of an 8 mm sample tube at 600 MHz showing: (A) signals from polarized pyridine in d4-methanol within a 1 mm slice, as compared to (B) those for unpolarised pyridine on the same sample after 128 averages; (C) analogous trace to (A) on a sample containing cylindrical rods of 1 mm internal diameter; (D) analogous trace to (B) on an unpolarised sample containing cylindrical rods of 1 mm internal diameter.

**sFig. 7.** 1H True FISP MRI images of an 8 mm sample tube at 600 MHz showing: (A) signals from polarized nicotinamide in d4-methanol within a 5 mm slice, as compared to (B) those for unpolarised nicotinamide on the same sample after 128 averages; (C) analogous trace to Fig 3 at 400 MHz for a 1 mm slice of polarized pyridine.

Fig. 7. Mixing field dependence of the longitudinal magnetization R, created for model complexes with AA'B spin topology in which the bound model substrate comprises a single spin.

Fig. 8. Mixing field dependence of the spin states created for model complexes with AA'BB', AA'BC, and ABCD spin topologies. The relative amplitudes for the zero quantum coherences $ZQ_x$ (black closed squares: ABCD; open squares: AA'BB'; and gray closed squares: AA'BC) and longitudinal two-spin order $Ri$, (black closed circles: ABCD; open circles: AA'BB'; and gray closed circles: AA'BC) are plotted as functions of the mixing field over the ranges of (a) 0--25 mT and (b) 25-250 mT. The relative amplitudes for the longitudinal magnetization $N$, (black closed squares: ABCD; open squares: AA'BB'; and gray closed squares: AA'BC) and $T$, (black closed circles: ABCD; open circles: AA'BB'; and gray closed circles: AA'BC) are plotted as functions of the mixing field over the ranges of (c) 0--25 mT and (d) 25-250 mT.

Fig. 9. The relative amplitudes of the longitudinal magnetization $k$, and $i$, associated with the isochronous substrate model plotted as a function of the mixing field. The plot shows the relative amplitudes after evolution of the free substrate I H nuclear spins in the mixing field, $R_z$ (open squares) and $i$, (open circles), and after transfer into the measurement field, k and $T$, (closed circles).

Fig. 10. The relative amplitudes of the zero quantum coherence $ZQ_x$ for the Anisochronous model plotted as a function of the mixing field after evolution at the mixing field (closed squares) and transfer to the measurement field (closed circles). For comparison, the amplitude of the same coherence for the complex at the point of dissociation is also included (open squares). The results are shown over the ranges of (a) 0-20 mT and (b) 20-250 mT. In (a), only one plot is apparent because the data sets for both the mixing field and complex produce exactly the same curve.

Fig. 11. The relative amplitudes of the longitudinal magnetization $R$: and $i$, for the Anisochronous model plotted as functions of the mixing field strength: at the mixing field (open squares, $R$, and closed squares, $i_z$) and after transfer to the measurement field (open circles, $R$, and closed circles, $i$). For comparison the same terms are included for the complex (open diamonds, $R_z$ and closed diamonds, $T_z$). The results are shown for the ranges of (a) 0-20 mT and (b) 20-250 mT.

## **Example 1**

**Preparation of active polarisation template [Ir(IMes)(H)$_2$(Py)$_3$][Cl] used in these studies.**

A small vial was charged with 2mg of [IrCl(IMes)(cod)], 5mg of the substrate to be polarized and 600 μl d4-methanol. The solution was transferred to a 5 mm NMR tube fitted with a Young's tap. The sample was thoroughly degassed by three freeze/pump/thaw cycles (NB. the sample is cooled to -78 °C rather than frozen to prevent cracking of the NMR tube upon warming). After warming the sample to room temperature, the NMR tube is pressurized to 3x10$^5$ Pa (3 atm) with p-H$_2$. The yellow solution turns pale yellow or colourless within approximately 5 minutes as the active species,

[Ir(IMes)(H)$_2$(sub)$_3$][Cl] forms.

## Characterization of [Ir(IMes)(H)$_2$(Py)$_3$][Cl]

The nature of the active catalyst is now exemplified by reference to the pyridine as the substrate.

Addition of excess pyridine (approx 25 equivalents) to a methanol solution of [IrCl(cod)(IMes)] resulted in the formation of a mixture of the starting material and the cationic complex [Ir(cod)(IMes)(Py)]$^+$. This cationic complex is supported by an anion which can take the form of an independent species such as Cl$^-$, BF$_4^-$ and PF$_6^-$ where the role is simply to balance the charge on the template.

When 3 atm H$_2$ is admitted to a sample tube containing this species, the colour of the solution changes over a period of 5 minutes from yellow to pale yellow. In this way, [Ir(IMes)(H)$_2$(Py)$_3$]$^+$ is formed in this *in-situ* reaction.

NMR spectroscopy (details below) revealed that the complex [Ir(IMes)(H)$_2$(Py)$_3$]$^+$ was formed in this case. When the sample was then degassed and $3 \times 10^5$ Pa (3 atm) of p-H$_2$ admitted to the sample, the NMR signature for free pyridine was polarized as shown in Figure 1

$\delta_H$(700 MHz; MeOD) $\delta$ 8.32 (s, br, 4H, ortho-H *trans*-Py), 8.04 (dt, 2H, *J* 5.0, 1.6, ortho-H *cis*-Py), 7.75 (t, 2 H *J* 6.8, para-H *trans*-Py), 7.66 (tt, 1H, *J* 7.6 and 1.5, para-H *cis*-Py), 7.10 (m, 6H meta-H *trans*-Py and Imidazole-CH), 6.95-6.97 (m, 2H, meta-H *cis*-Py), 6.65 (s, 4H, Mesitylene CH), 2.18 (s, 6H, Mesitylene *para*-CH$_3$), 2.04 (t, 12 H *J* 4.8, mesitylene *ortho*-CH$_3$).

## Synthesis of [IrCl(COD)(IMes)]

This complex was synthesized by a new method.

Under an atmosphere of N$_2$, a schlenk was charged with [Ir(COD)($\mu$-OMe)]$_2$ (345 mg, 0.517 mmol) and IMes.HCl (175 mg, 1.044 mmol). Dry acetone (15 mL) was added to the Schlenk by cannula. The bright yellow solution was stirred at room temperature for 30 minutes, after which time it had turned a golden yellow colour. The solvent was removed in vacuo to give the crude product as a golden yellow solid. The solid product was purified by flash column chromatography on silica in 9:1 dichloromethane:acetone. Yield 600mg, 90 %.

1H NMR: (500 Mhz, CDCl$_3$) $\delta$ 7.04 (s, 2H, Ar H), 7.00 (s, 2H, Ar H), 6.98 (s, 2H - NCHCHN-), 4.18 (m, 2H, COD), 3.00 (m, 2H, COD), 2.19 (s, 12H, Ar 2-Me), 1.79-1.63 (m, 4H COD CH$_2$), 1.57 (s, 6H, Ar-4-Me), 1.41-1.32 (m, 2H COD CH$_2$), 1.31-1.24 (m, 2H, COD CH$_2$)

LIFDI MS: *m/z* = 605.2489 ([IrCl(IMes)(COD)]$^+$), 305.1219 (IMes$^+$).

We note that IMes (1,3-dimesityl-imidazol-2-ylidene) is simply a representative example of an N-heterocyclic carbene. This ligand type acts as a strong sigma donor and therefore promotes the assembly of the template to support both H$_2$ and the substrate. We note that the efficacy of this template can be controlled by changing the substituents on the carbene. For example the groups could be changed from 2,4,6 Me, to 2,6 iPr and to 2,6 Me. In this way specificity and efficacy might be addressed. We further note that the NHC backbone can be saturated in order to provide a further opportunity to impart control.

We further note that supported variants of these N-heterocyclic carbene complexes can be prepared. For example by using 1-methyl-3-(4-vinylbenzyl)imidazolium hexafluorophosphate and copolymerising it with styrene and divinylbenzene. When this material is then exposed to the iridium precursor, a polymer supported template is generated. In this was we ensure that the polarised materials and their polarisation template can be readily separated.

## Materials and Methods

**Active complex characterisation:** We have taken a sample of a complex [Ir(COD)(PCy$_3$)(Py)]BF$_4$ (1) (COD = cycloocta-1,5-diene, Cy = cyclohexyl, Py = pyridine) (1) and dissolved it in d$_4$-methanol at 300 K. The substrate (e.g. pyridine, 5$\mu$L) was then added to the solution which was transferred to a 5mm Young's tap NMR tube. The solution was degassed and the NMR tube pressurised with para-H, (3 atm). When the resultant reaction is monitored by $^1$H NMR spectroscopy, the hydride containing complex [Ir(H)$_2$(PCy$_3$)(Py)$_3$]BF$_4$ 2 in Scheme 1 is detected. The associated $^1$H NMR spectrum of the hydride region of this spectrum is shown in sFig. 1. The hydride signal for complex 2 shows no apparent signal enhancement and appears as a simple doublet at $\delta$-23.51 (JPH = 24.3 Hz). Upon $^{31}$P decoupling, the single resonance at $\delta$-23.51 for complex 2 simplifies to a singlet. Since this hydride resonance possesses only a small coupling to a single $^{31}$P centre the phosphine must be arranged *cis* to the hydride ligands. The $^{31}$P chemical shift for complex 2 was determined through a $^1$H-$^{31}$P HMQC experiment and appears at $\delta$13.1, although in the hydride coupled 1-dimensional $^{31}$P spectrum the $^{31}$P signal splits into a triplet thereby confirming its origin in a dihydride.

When $^{15}$N-labelled pyridine is used as the substrate, the hydride resonance for complex 2 becomes PHIP enhanced with an added coupling of 21.5 Hz and dominates the hydride region of the spectrum. This enhancement is the result of the introduction of the isotopic label which transforms the magnetic environment of the symmetrical dihydride complex from a chemically equivalent and magnetically equivalent situation to a magnetically inequivalent situation by virtue of the new second order AA'XX' spin system. The appearance of this resonance therefore confirms the identity of 2 as a dihydride complex where both hydride ligands lie *trans* to pyridine. The $^{15}$N chemical shift data for the pyridine ligand resonances for the group *trans* to the hydride ligands in 2 was easily located in a $^1$H-$^{15}$N HMQC experiment at -48.3 ppm. In addition, when a $^1$H-$^{31}$P HMQC spectrum is recorded of these $^{15}$N-labelled complex, the $^{31}$P signal of 2 now appears as a doublet with a splitting of 51 Hz which demonstrates the coordination site *trans* to phosphine is occupied by an additional pyridine ligand. On the basis of this information, complex 2 corresponds to the product *fac,cis*-[Ir(PCy$_3$)(Py)$_3$(H)$_2$]BF$_4$. This species is similar to the known complex [Ir(P[i]Pr$_3$)(MeCN)$_3$(H)$_2$][+] (2).

**Ligand Exchange:** When a sample of complex 2 is prepared and the aromatic region of the $^1$H NMR spectrum examined, two sets of resonances due to the three pyridine ligands with relative populations of 1:2 are detected for complex 2. Interestingly, when a 2D-EXSY NMR experiment is recorded to monitor the ability of these ligands to undergo exchange, cross peaks indicative of exchange into free pyridine were seen for the pyridine site that is *trans* to the hydride ligand. Specifically, significant transfer from the δ 8.94 resonance for the *ortho-pyridine* proton for the ligand *trans* to hydride was observed into the free pyridine signal at δ 8.54 with short mixing times. In contrast the δ 8.62 signal which arises for the *artha-proton* on the pyridine that is *trans* to phosphine failed to show such a cross peak. It would therefore seem that the *in-situ* transfer of *parahydrogen* induced polarization to free pyridine (or the specified substrate) occurs through ligand exchange in 2 (or its analogue). In support of this, we note the resonances for the two equivalent bound pyridine ligands of complex 2 also share this effect.

**Spontaneous Polarisation Transfer:** The most noticeable features of the NMR spectra we describe are seen when a sample is first observed after reacting with para-H$_2$ in low magnetic field outside the main NMR magnet. This effect is illustrated in sFig. 2 and sFig. 3 which show a series of single scan $^1$H NMR spectra for a range of substrates before (top trace) and after (bottom trace) polarization.

**$^{13}$C Signal to Noise Determination:** A $^{13}$C NMR spectrum of polarized pyridine was acquired with one scan using a 90° pulse followed by a short delay for refocusing and 1 second acquisition time during which $^1$H decoupling was applied. The signal to noise ratios of the resonances for the meta and para carbons were determined to be 91.76 and 25.75 respectively. After allowing for full relaxation of the polarization, a $^{13}$C NMR spectrum of the same sample was acquired using a 90° pulse with inverse gated decoupling and a 20 second recycle delay. After 10,240 scans were collected the signal to noise ratios for the meta and para carbon resonances of pyridine were determined to be 11.28 and 7.75 respectively. This result reflects an enhancement of 823 fold for the meta carbon signal on the basis that the signal to noise from 1 scan equates to that of 677,329 scans in the unpolarised sample.

**Theoretical Description:** When para-H$_2$ adds to a metal complex which also contains a substrate, the para-H$_2$ and substrate become spin-spin coupled. By employing the correct molecular design, it is possible to generate the second order spin system AA'XY at high field where AA' are para-H$_2$ derived spins and the XY spins correspond to components of the model substrate. At low field, however, the scalar coupling between the spins will be strong relative to the chemical shift differences and the spin system becomes AA'BC. In this case polarisation can be transferred from the AA' spins to the substrate through the J- coupling network.

Under zero field conditions, polarisation is transferred in such a way that pseudo-singlet states are created between all pairs of proton coupled spins within the model substrate, i.e., labelling parahydrogen as I and S spins and the substrate as R and T, would create;

$$a(I_xS_x + I_yS_y + I_zS_z) + b(I_xR_x + I_yR_y + I_zR_z) + c(I_xT_x + I_yT_y + I_zT_z)$$
$$+ d(S_xR_x + S_yR_y + S_zR_z) + e(S_xT_x + S_yT_y + S_zT_z) + f(R_xT_x + R_yT_y + R_zT_z),$$

where the amplitudes a, b, c, d, e and f depend on the values of the interspin coupling constants.

In contrast to hydrogenation reactions, where a strong permanent covalent bond is established between two carbon and hydrogen atoms, our metal complex is labile and dissociates both the para-H$_2$ and the substrate. At this point, the terms which involve a hydrogen spin from what was para-H$_2$, and a spin from the substrate are destroyed. Consequently, for this four spin model, with transfer at zero field, only the two terms below remain:

$$\mathrm{a}(I_x S_x + I_y S_y + I_z S_z) + \mathrm{f}(R_x T_x + R_y T_y + R_z T_z).$$

sequences whose parameters were: *TE/TR = 4.0/2.0* ms, BW = 150 kHz, FOV = 20 mm x 20 mm, matrix = 128 x 128 and the slice thickness was 20.0 mm. This means that the inherent sensitivity associated with the two measurements differs by a factor of 160.

Further signal to noise calculations for imaging experiments are now illustrated for data collected at 600.12 MHz (14.1 T). It should be noted that due to the physical size of the magnet, it takes much longer to introduce the sample into the spectrometer for measurement and any enhancement factors will be substantially less than those recorded on the 9.4 T system. To avoid signal reduction due to partial volume effects the central part of the image was used to measure the signal while the noise was measured in the top right hand corner of the image.

For these studies, a sample of 40 ~l pyridine and 6 mg polarisation transfer catalyst in 2 ml MeOD was employed and a True FISP image collected whose parameters were: TR 4.0 ms, TE 2.0 ms, FA 60°, MTX 128 x 128, and the FOV was 2 cm x 2 cm. A slice thickness of 1mm gave SIN values for the polarized system with a single scan average of S/N: 18.10, as illustrated in sFig 6(a). The corresponding unpolarised system yielded a S/N value of 1.27 after a single scan but this increased to 7.30, as shown in sFig. 6b after 128 averages were recorded.

Addition of glass tubes of 1 mm internal diameter gives a demonstration of the resolution achievable when exciting a 5 mm slice. No signal was observed when using a 1 mm slice for the unpolarised system. A 5 mm slice examined using the polarized system and a 1 scan average, is shown in sFig. 6c, with the corresponding unpolarised trace collected

We note the term *RxTx + RyTy + RzT$_z$* is unaffected by dipole-dipole interactions that lead to longitudinal relaxation and that it can be associated with a long lived magnetic state. Upon introducing the sample into the spectrometer, the remaining para-Hi does not influence this process further but the remaining terms evolve appropriately under the effects of chemical shift and scalar coupling with the longitudinal two spin order term f*(RzTz)* always remaining.

When the polarization transfer from para-Hi is carried out at a low field rather than zero field, where spin rotations due to the J coupling are comparable to the chemical shift evolution then the symmetry of the pseudo-singlet state is broken. Consequently the amplitude of the longitudinal two spin order is no longer equal to that of the zero quantum term and the description of the magnetisation becomes more complicated. In fact, the interaction of the chemical shift and scalar couplings together results in zero quantum coherences being transformed into z-magnetisation in the form $R_z - T_z$. In sFig. 4 and sFig. 5 the amplitudes of the substrate spin states, $R_z T_z$*(pale* blue), $R_x T_x + R_y T_y$ (green), $R_z$ (blue) and $T_z$ (red) are plotted against the field at which the polarization transfer and subsequent dissociation was carried out. In these results B and C are hydrogen atoms. The coupling between B and the two hydrogen's from para-$H_2$ were 3 and 1 Hz respectively, representing the asymmetry due to *cis* and *trans* coupling across the metal in the complex. The coupling between B and C was set at 7 Hz while the coupling between C and the para-$H_2$ was vanishingly small. The coupling between the AA' spins was set to 8 Hz.

From sFig. 4, it is clear that the amplitude of the spin states is sensitive to the polarisation transfer field over the range 0 to 0.015 T. At higher field strengths (0.015 - 0.2 T) as shown in sFig. 5, the spins state amplitudes change more smoothly until at approximately 0.2 T no polarization is transferred from para-H, to the substrate. We note that the quoted employing 128 averages being shown in sFig. 6d. Only a very weak signal can be discerned in this latter image.

Images for a sample of 20 mg nicotinamide and 4 mg polarisation transfer catalyst in 2 ml MeOD were also collected using the True FISP sequence with TR 4.0 ms, TE 2.0 ms, FA 60°, MTX 128 x 128 and a FOV of 2 cm x 2 cm. This resulted in a signal to noise values for a 5 mm slice for the polarized system with a 1 scan average of (S/N) 9.20 as shown in sFig. 7a. The corresponding unpolarised system produced a S/N value for the corresponding 1 scan average of 2.05, which scaled to 7.98 after 128 averages, as shown in sFig. 7b. For a 2.5 mm slice, the polarized system (single average) gave a S/N: 9.08 while the unpolarised system (single average) yielded a S/N of 1.95 which scaled with 128 averages to a S/N: 3.84. These data establish that the hyperpolarisation process facilitates the collection of MR images that would otherwise require substantially more averaging. Image collection using slice thicknesses of 1 mm and 0.5 mm are easily achievable using this technique on the systems described. This is exemplified by the images in the manuscript (we note sFig. 7c corresponds to a single averaged, 1 mm image, of the same sample used in Fig 3 of the main text). Comparison of data presented in the main manuscript with that recorded using a Boltzmann derived signal proved to be impractical due to the time required to collect such images.

High Resolution NMR Methods: NMR measurements were made on a Bruker DRX400 CH at 400.13 MHz, $^{31}$P at 161 MHz, [15]$_N$ at 40 MHz), a Bruker AV500 CH at 500.22 MHz, $^{31}$P at 202.50 MHz, $^{15}$N at 50.69 MHz) a Bruker widebore AV600 CH at 600.12 MHz, $^{31}$P at 242.93 MHz, $^{15}$N at 60.81 MHz) and a Bruker AV700 CH at 700.12 MHz, $^{31}$P at

283.36 amplitudes in sFig. 4 and sFig. 5 are indicative of product states that will produce observable magnetisation with an enhancement greater than four orders of magnitude. The introduction of the sample into the strong magnetic field for observation will again lead to further evolution of the magnetisation.

**Control Experiments:** Complexes such as **1** are known to catalyze the deuterium labelling of organic compounds. There remains therefore the possibility that the observed polarizations are due to the chemical exchange of pyridine H-atoms with those derived from para-H$_2$. We discount this possibility on the basis of $^2$H-labeling studies and the obvious test that pyridine-d$_5$ itself does not yield $^1$H-polarisations. Thus, when d$_5$-pyridine is employed as the substrate, very limited net signal enhancements are observed due to the residual protons in the d$_4$-H$_1$-pyridine even over a period of several days. Complex 2 however does undergo exchange its hydride ligands with the deuterium in the methanol solvent.

**Imaging Sequences: Imaging Sequences:** The presented images were recorded using a Bruker DSX 400 Spectrometer ($^1$H frequency is 400.12 MHz, 9.4 T) or a Bruker wide-bore AV600 ($^1$H at 600.12 MHz, **14.1** T). The systems are equipped for micro-imaging with Great40 gradient systems (Calibrated maximum 1.04 T / m). All images were acquired using the true-FISP pulse sequence on a J3C;iH dual-tuned coil or a $^1$H coil.

In the manuscript two traces recorded at 9.4 T are presented. For the polarized situation the single average image was collected with a sequence whose parameters were: *TE/TR = 4.0/2.0* ms, FA = 60°, BW = 150 kHz, FOV = 20 mm x 20 mm, matrix = 256 x 256 and the slice thickness was 0.5 mm. For the control images the images were collected using a MHz, $^{15}$N at 70.93 MHz) NMR spectrometer. NMR samples were prepared in 5 mm Young's tapped NMR tubes. Typical samples contained 1 mg of the complex dissolved in CD$_3$OD (600 μL) with the other reagents being added by micro-syringe. Samples were then degassed by three cycles of pumping under high vacuum at -78°C followed by vigorously shaking the tube. Samples were then pressurised by 3.5 atm. para-H$_2$ prior to the commencement of NMR measurement. para-H$_2$ was prepared by cooling the gas to 20K over activated charcoal in the apparatus described previously (3).

## Results and Discussion

It is worthwhile considering the polarised spin state obtained due to spontaneous polarisation transfer from *para*hydrogen to the $^1$H nucleus on a model substrate comprising only a single spin. In such a situation, the complex would have a spin topology of AA'B if the *para*hydrogen nuclei are isochronous or ABC if they are anisochronous. As it transpires, the results obtained for these two topologies are almost identical. In general, using the product operator notation, the three-spin complex will evolve into

$$
\begin{aligned}
&a_{ZZ}\left(\hat{I}_z\hat{S}_z\right)+a_{ZQ}\left(\hat{I}_x\hat{S}_x+\hat{I}_y\hat{S}_y\right)\ +a_Z\left(\hat{I}_z-\hat{S}_z\right)+\\
&b_{ZZ}\left(\hat{I}_z\hat{R}_z\right)+b_{ZQ}\left(\hat{I}_x\hat{R}_x+\hat{I}_y\hat{R}_y\right)\ +b_Z\left(\hat{I}_z-\hat{R}_z\right)+\\
&c_{ZZ}\left(\hat{S}_z\hat{R}_z\right)+c_{ZQ}\left(\hat{S}_x\hat{R}_x+\hat{S}_y\hat{R}_y\right)+c_Z\left(\hat{S}_z-\hat{R}_z\right)
\end{aligned}
\tag{29}
$$

$$+ \text{zero quantum terms involving all three spins.}$$

under strong scalar coupling and chemical shift.

When the substrate dissociates from the complex, any terms involving nuclei originating from both the *para*hydrogen and the substrate will be lost, thus the only surviving term for the polarised free substrate nucleus is $-(b_z + c_z)$ $\hat{R}_z$. The dependence of the $\hat{R}_z$ amplitude on the strength of the mixing field is shown in Fig. 7. This $\hat{R}_z$ term is generated by the evolution of the two-spin zero quantum terms $\hat{I}_x\hat{R}_x + \hat{I}_y\hat{R}_y$ and $\hat{S}_x\hat{R}_x + \hat{S}_y\hat{R}_y$ under the concerted effects of strong scalar coupling and the chemical shift difference between the spin pairs *IR* and *SR*[10,17]. At zero field, there is no chemical shift evolution and the amplitudes $b_z$ and $c_z$ are zero. That is to say, there is no polarisation of the free substrate. At higher fields, the spin system evolves under conditions that can be described through the weak coupling approximation. In this regime the $ZQ_x$ terms, $\hat{I}_x\hat{R}_x + \hat{I}_y\hat{R}_y$ and $\hat{S}_x\hat{R}_x + \hat{S}_y\hat{R}_y$ evolve into their conjugate $ZQ_y$ terms $\hat{I}_y\hat{R}_x - \hat{I}_x\hat{R}_y$ and $\hat{S}_y\hat{R}_x - \hat{S}_x\hat{R}_y$ and do not evolve under scalar coupling at all. As in the zero field case, no $\hat{R}_z$ polarisation is created. In between these two extremes, longitudinal magnetisation, $\hat{R}_z$, is created with the level of the polarisation being dependent on the balance between the scalar coupling and the chemical shift evolution. In this model, the maximum amplitude for $\hat{R}_z$ occurs when the chemical shift difference between each of the *para*hydrogen $^1$H nuclei and the substrate $^1$H nucleus is close to the scalar coupling between the two *para*hydrogen $^1$H nuclei. More surprisingly, the field at which the maximum polarisation occurs is relatively insensitive to the coupling between the *para*hydrogen $^1$H nuclei and the substrate $^1$H nucleus.

**Generation of free substrate with polarisation transfer at zero field**

In the absence of chemical shift evolution at zero field, spin polarisation must be propagated across the whole spin system through the scalar coupling network only. Intuitively, this is perhaps a little surprising, since the singlet state for a single pair of spins commutes with the scalar coupling Hamiltonian. However, when the same singlet state is introduced into a larger spin topology, it commutes only with the full scalar coupling Hamiltonian when all the couplings in the network are equal. We observe here that it is the fact that the scalar couplings are *not* identical which forms the very basis of the polarisation transfer mechanism.

During the lifetime of the complex, the four-spin system evolves into a

$$(\hat{I}_x\hat{S}_x + \hat{I}_y\hat{S}_y + \hat{I}_z\hat{S}_z) + \mathrm{b}(\hat{I}_x\hat{R}_x + \hat{I}_y\hat{R}_y + \hat{I}_z\hat{R}_z) + \mathrm{c}(\hat{I}_x\hat{T}_x + \hat{I}_y\hat{T}_y + \hat{I}_z\hat{T}_z)$$

$$+ \mathrm{d}(\hat{S}_x\hat{R}_x + \hat{S}_y\hat{R}_y + \hat{S}_z\hat{R}_z) + \mathrm{e}(\hat{S}_x\hat{T}_x + \hat{S}_y\hat{T}_y + \hat{S}_z\hat{T}_z) + \mathrm{f}(\hat{R}_x\hat{T}_x + \hat{R}_y\hat{T}_y + \hat{R}_z\hat{T}_z) \qquad (30)$$

+ terms involving three and four spins.

In Eq. 30, the amplitudes *a, b, c, d, e, f* and those related to the three- and four-spin terms are determined by the specific values of all the scalar couplings within the complex. They are also time averaged over the interval $E_1$. Note that amplitudes, *a* and *f*, used in Eq. 29 should not be confused with the time-dependent amplitudes and the distribution function, $f_r(\bar{t}_d| t_1)$. Of particular note is the fact that, due to the absence of chemical shift evolution at zero field, polarisation is transferred to states involving two, three and four spins, but not to single spin states. In addition, at zero field, all of the two-spin terms, including the two former parahydrogen $^1$H nuclei, evolve into spherically-symmetric, pseudo-singlet states.

When the complex dissociates into hydrogen and polarised free substrate, the only couplings that remain are those between the original hydrogen $^1$H nuclei, *I* and *S,* and between the two $^1$H nuclear spins of the substrate, *R* and *T.*

The free substrate will evolve for a time, $t_f$, at zero field, before being inserted into the magnet. Substituting the initial boundary conditions $a_{RzTz} = a_{ZQx} = f$, $a_{ZQy} = 0$, $a_{Rz} = 0$, $a_{Tz} = 0$ and $\delta = 0$ into Eq. 7 gives $f(2\hat{R}_z\hat{T}_z + ZQ_x)$ as the spin state for the polarised free substrate at the end of interval $E_2$.

If the free substrate is isochronous, i.e. $B_2$, then, as we have seen, the spin system does not evolve during interval $E_3$ and the final state is $f(2\hat{R}_z\hat{T}_z + ZQ_x)$. When the substrate is anisochronous, i.e. BC, the spin system evolves and, after time averaging, gives a final spin state according to Eq. 27:

$$\bar{\rho}_m(t_f) = a_{RzTz}2\hat{R}_z\hat{T}_z + c(t_f)\hat{R}_z + d(t_f)\hat{T}_z \qquad (27).$$

Substituting $a_{RzTz} = f$, $c(t_f) = a_{Rz} = 0$ and $d(t_f) = a_{Tz} = 0$ into Eq. 27, we find the time-averaged spin state at the end of interval $E_3$ is $f2\hat{R}_z\hat{T}_z$.

There is, therefore, a difference between the two spin topologies of the $B_2$ and BC spin systems at this point. This is most readily visualised by the fact that the magnetisation in the Anisochronous model is predicted to be observed with a non-90° pulse producing a familiar antiphase doublet. On the other hand, the magnetisation in the Isochronous model cannot be observed at all because the singlet state is invariant to rotation. This term can, therefore, only be observed in practice by breaking the symmetry of the Isochronous substrate, a process that is fully reflected in hydrogenative PHIP.

**Evolution of the 4-spin complex at non-zero field**

Conceptually, there is no difference in the ABCD type complexes formed from the Isochronous and Anisochronous model substrates, though differences in the spins states of the substrates do exist after their dissociation. As such, in the context of the evolution of the complexes, we only show results for AA'BB', AA'BC situations involving the Isochronous, $B_2$, substrate and the ABCD complex involving the Anisochronous substrate.

In a non-zero field, the nuclear spins in these complexes will evolve under both chemical shift and scalar coupling. At very low fields, the rotations due to the chemical shift are of a similar order of magnitude as those due to the scalar coupling. The combined effect of the scalar coupling and chemical shift is to interconvert any $ZQ_x$ coherence, $\hat{I}_x\hat{S}_x + \hat{I}_y\hat{S}_y$, into longitudinal magnetisation of the type $\hat{I}_z\text{-}\hat{S}_z$ In general, at the point of dissociation, the complex will therefore be in a state

$$a_{ZZ}\left(2\hat{I}_z\hat{S}_z\right)+a_{ZQx}\left(2\hat{I}_x\hat{S}_x+2\hat{I}_y\hat{S}_y\right)+a_z\left(\hat{I}_z-\hat{S}_z\right)+$$
$$b_{ZZ}\left(2\hat{I}_z\hat{R}_z\right)+b_{ZQx}\left(2\hat{I}_x\hat{R}_x+2\hat{I}_y\hat{R}_y\right)+b_z\left(\hat{I}_z-\hat{R}_z\right)+$$
$$c_{ZZ}\left(2\hat{I}_z\hat{T}_z\right)+c_{ZQx}\left(2\hat{I}_x\hat{T}_x+2\hat{I}_y\hat{T}_y\right)+c_z\left(\hat{I}_z-\hat{T}_z\right)+$$
$$d_{ZZ}\left(2\hat{S}_z\hat{R}_z\right)+d_{ZQx}\left(2\hat{S}_x\hat{R}_x+2\hat{S}_y\hat{R}_y\right)+d_z\left(\hat{S}_z-\hat{R}_z\right)+$$
$$e_{ZZ}\left(2\hat{S}_z\hat{T}_z\right)+e_{ZQx}\left(2\hat{S}_x\hat{T}_x+2\hat{S}_y\hat{T}_y\right)+e_z\left(\hat{S}_z-\hat{T}_z\right)+$$
$$f_{ZZ}\left(2\hat{R}_z\hat{T}_z\right)+f_{ZQx}\left(2\hat{R}_x\hat{T}_x+2\hat{R}_y\hat{T}_y\right)+f_z\left(\hat{R}_z-\hat{T}_z\right)$$

+ terms of higher spin order (31)

As in the case of zero field, the values of the amplitudes in Eq. 31 depend upon the magnitudes of the scalar couplings and the lifetime of the complex. In non-zero field, however, the amplitudes also depend upon the differences in the chemical shifts of the spins and this introduces complex field dependencies. The mixing field dependences of the amplitudes, $f_{ZZ}$, $f_{ZQx}$, $(f_z - b_z - d_z)$ and, $-(c_z + e_z + f_z)$, associated with the spin terms $2\hat{R}_z\hat{T}_z$, $(2\hat{R}_x\hat{T}_x + 2\hat{R}_y\hat{T}_y)$, $\hat{R}_z$ and $\hat{T}_z$ are shown in Fig. 8 for the three spin topologies, AA'BB', AA'BC and ABCD of the complex. The amplitudes of the spin terms $2\hat{R}_z\hat{T}_z$, and $(2\hat{R}_x\hat{T}_x + 2\hat{R}_y\hat{T}_y)$ are shown in Fig. 8(a) for the range of polarisation field strengths 0-25 mT and Fig. 8(b) for the range of polarisation field strengths 25-250 mT. The $\hat{R}_z$ and $\hat{T}_z$ amplitudes are shown in Fig. 8(c) (0-25 mT) and Fig. 8(d) (25-250 mT). Amplitudes are reported as fractions of the maximum $2I_zS_z$ amplitude obtainable from *para*hydrogen in a traditional PASADENA experiment involving hydrogenation.[3,4,10] In practice, $2\hat{I}_z\hat{S}_z$ amplitude enhancements of 31,000 times have been observed for *para*hydrogen addition reactions at 295 K in a field of 9.4 T.

The three spin topologies show very similar behaviour, especially at the lowest field strengths 0 to 25 mT, and we now discuss the field dependency generally. Below 15 mT the subtle balance between the scalar coupling and the chemical shift evolution of the nuclear spins produces a complex behaviour for each of the resultant amplitudes. At certain fields, the chemical shift difference between spin pairs combines with the scalar coupling to favour polarisation transfer across them. For example, polarisation transfer to the substrate $^1$H nuclei in the form of a singlet state appears to be favoured at 0 mT, whereas mixed states are favoured at 5 mT and 10 mT (Fig. 8(a)). As discussed above, at zero field, only the scalar coupling part of the Hamiltonian propagates polarisation across the network of spins. It is clear that as the mixing field is increased from zero, even by a small amount, the chemical shift contribution to the Hamiltonian breaks up the pseudo-singlet states between pairs of spins because the $ZQ_x$ term evolves. The evolution of this term under the concerted effects of the scalar coupling and the chemical shift, however, generates longitudinal magnetisation in the form of $\hat{R}_z$ and $\hat{T}_z$. The similarity of the amplitudes within both Figs. 8(a) and 8(c) highlights the fact that the form of the field dependence at these field strengths is dominated by the scalar coupling *and* by the chemical shift difference between the spins originating from the *para*hydrogen and those from the substrate. In fact, for the AA'BB' model there is no chemical shift difference between A and A' and between B and B' and subsequently there is no chemical shift evolution of the zero quantum states, $2\hat{I}_x\hat{S}_x + 2\hat{I}_y\hat{S}_y$ and $2\hat{R}_x\hat{T}_x + 2\hat{R}_y\hat{T}_y$. The absence of chemical shift evolution means that the amplitudes $a_z$ and $f_z$ in Eqn. 31 will be zero.

This evolution is, of course, a dynamic process that involves the two-spin $ZQ_y$ coherence (e.g. $2\hat{R}_y\hat{T}_x - 2\hat{R}_x\hat{T}_y$) as well as three- and four-spin terms. However, due to the stochastic nature of the complex formation and dissociation, the only substrate spin terms that survive dissociation are $2\hat{R}_z\hat{T}_z$, $ZQ_x$, $\hat{R}_z$ and $\hat{T}_z$. Some three- and four-spin states are also generated, but are lost when the complex dissociates. It should be noted, however, that in any substrate model containing more than two spins, the terms involving spin orders greater than two, in which all the spins belong to the substrate, would not be lost after dissociation.

As the field is increased, above 15 mT, the chemical shift contribution becomes stronger and the amplitude of the $2\hat{R}_z\hat{T}_z$ term becomes smaller (Fig. 8(a)). This occurs because the scalar coupling component that mixes this term with the $ZQ_x$ term, which in turn interconverts with the $\hat{R}_z$ and $\hat{T}_z$ terms through the transitory $ZQ_y$ coherences, is less efficient. In fact, for any pair of spins, say R and T, the maximum amplitude for the term $\hat{R}_z - \hat{T}_z$ occurs when the chemical shift difference between the spins matches the scalar coupling of the *para*hydrogen spins. However, the amplitude of the $ZQ_x$ terms are dependent on the asymmetry of the scalar coupling network. The chemical shift difference between a $^1$H nuclear spin originating from *para*hydrogen and one originating from the bound substrate is considerably larger than the chemical shift difference between the two substrate $^1$H nuclear spins. Thus, these former chemical shift differences match the scalar coupling at a lower field than the latter. In fact, in the AA'BB' complex the latter is actually zero. It is clear that the below 15 mT, all spin pairs with non-zero chemical shift differences are contributing to all spin states.

In contrast, at higher mixing fields, the chemical shift difference between *para*hydrogen spins and substrate spins are sufficiently large so that at 25 mT the spin pairs *IR, IT, SR* and *ST* evolve under conditions approaching the weak coupling regime, whilst the substrate spins pair *RT* can still be considered to be strongly coupled.

The difference between the AA'BB' topology and the other topologies become more apparent at field strengths between 25 mT and 250 mT. At mixing fields greater than 20 mT, no polarisation is transferred to the longitudinal two-spin order of the substrate spins *R* and *T*. Hence the amplitude of the $ZQ_x$ term begins to decrease at larger mixing fields as the chemical shift differences between the A and B, and C and D spins in the ABCD topology and between the B and C spins in the AA'BC topology become larger. The interconversion of the $ZQ_x$ coherence of the R and T spins and the $\hat{R}_z$ and $\hat{T}_z$ states dominates the shape of the field dependence curve as the other spin pairs tend toward weak coupling regimes (Fig. 8(b) and Fig 8(d)). In contrast, the $ZQ_x$ term in the AA'BB' topology reaches a plateau, confirming that the changes in the amplitudes of the $ZQ_x$, $\hat{R}_z$ and $\hat{T}_z$ states in the AA'BC and ABCD topologies are the results of the respective chemical shift differences of the substrate nuclei. Above 50 mT, the amplitudes $b_z$, $c_z$, $d_z$ and $e_z$ tend to zero as the appropriate spin pairs tend to the weak coupling regime and the term $f_z$ dominates when *spin R* and *T* have different chemicals shifts (Fig. 8(d)). In the AA'BB' topology $f_z$ is zero at all mixing fields because the chemical shift difference between *R* and *T* is zero (Fig. 8(d)).

It becomes apparent that at low field strength, the field-dependence of the polarisation transfer crucially depends on the balance between the full scalar coupling and chemical shift components of the Hamiltonian and that the details of the field-dependence need to be explored using the full density matrix calculation.

Evolution of the free substrate in the Isochronous model

After dissociation, there is a period of time in which the spins of the substrate $^1$H nuclei continue to evolve in the mixing field. To some extent this time is under the control of the experimentalist. This may range from the briefest time that is physically possible in order to transfer the sample from the mixing field into the measurement field, to much longer times where, for example, investigations into the existence of long-lived states may be completed. It is assumed that even the shortest of these times may be considered long with respect to the spin dynamics and relatively short with respect to relaxation, such that the loss of polarisation due to relaxation would be minimal. In this work we are interested in the mechanisms of polarisation transfer and relaxation processes have not been considered.

Eq. 28 describes the time-averaged amplitudes at the end of interval $E_2$

$$\bar{a} = \frac{2a_{ZQx}J^2 + \delta(a_{Rz} - a_{Tz})}{2(J^2 + \delta^2)}$$

$$\bar{b} = 0$$

$$\bar{c} = \frac{2J\delta a_{ZQx} + (2\delta^2 + J^2)a_{Rz} + J^2 a_{Tz}}{2(J^2 + \delta^2)}$$

$$\bar{d} = \frac{-2J\delta a_{ZQx} + (2\delta^2 + J^2)a_{Tz} + J^2 a_{Rz}}{2(J^2 + \delta^2)} \tag{28}$$

The initial boundary conditions are given as, $a_{RzTz} = a_{RzTz}$, since $2\hat{R}_z\hat{T}_z$ does not evolve under the two-spin Hamiltonian and, from Eq. 31, $a_{ZQx} = f_{ZQx}$, $a_{Rz} = f_z - b_z - d_z$ and $a_{Tz} = -f_z - c_z - e_z$. Substituting these into Eq. 28 and setting $\delta = 0$ for the Isochronous substrate gives

$$\bar{a} = f_{ZQx}$$

$$\bar{b} = 0$$

$$\bar{c} = \bar{d} = \frac{-(b_z + c_z + d_z + e_z)}{2}. \tag{32}$$

This result clearly shows how the spin state depends upon the boundary states generated during the evolution of the complex. What is notable is that the amplitude of $\hat{R}_z$ and $\hat{T}_z$ do not depend on the amplitude $f_z$, regardless of the topology of the complex.

When the mixing field is zero, $a_{ZQx} = a_{RzTz} = f_{ZZ}$ and $b_z = c_z = d_z = e_z = 0$ and the spin pair exists in a pure singlet state. This does not evolve either at the mixing field or during the transition to the measurement field because the chemical shift difference between the two spins is zero. In fact, even when in the measurement field, the hyperpolarisation of this spin pair would not be detectable due to the spherical symmetry of the singlet state which makes it invariant to rotation by an RF-pulse.

At non-zero field, the longitudinal two-spin order term, $2\hat{R}_z\hat{T}_z$, and the $ZQ_x$ do not evolve at all as can be seen from Eq. 32. In effect, the amplitudes of these two terms at the measurement field are determined by the evolution of the spins while they were part of the complex, as shown in Figs. 8(a) and 8(b). The only terms that actually evolve over interval $E_2$ are $\hat{R}_z$ and $\hat{T}_z$. Under the symmetry of the B$_2$ topology, the two amplitudes become equal, as shown in Fig 9 and Eq. 32. As $b_z$, $c_z$, $d_z$ and $e_z$ tend to zero at larger mixing fields, the amplitudes of $\hat{R}_z$ and $\hat{T}_z$ also tend to zero. Since $\delta = 0$ for the Isochronous substrate, there is no further evolution as the field strength is increased to the measurement field.

Unlike the singlet state, where the amplitudes are equal, the state $f_{zz} 2\hat{R}_z\hat{T}_z + f_{ZQx}(ZQ)_x$ can be interrogated using a non-90° RF-pulse with the resulting signal amplitude being proportional to $f_{zz} - f_{ZQx}$. The signal arising from these terms would be antiphase, corresponding to the single quantum coherences of the types $2\hat{R}_z\hat{T}_x$ and $2\hat{R}_x\hat{T}_z$. In addition, and superimposed on the antiphase signals, there will be in-phase signals associated with $\hat{R}_x$ and $\hat{T}_x$ originating from the $\hat{R}_z$ and $\hat{T}_z$ terms. Interrogation of the spin state with a 90° RF-pulse will generate a spectrum containing only the in-phase signals originating from $\hat{R}_z$ and $\hat{T}_z$. Above 20 mT, however, the only non-zero amplitude will be that of the $ZQ_x$, resulting in a simpler antiphase spectrum after a non-90° RF-pulse.

Evolution of the free substrate in the Anisochronous model

Although there are small differences in the amplitudes of the spin states for the complexes with AA'BC and ABCD topologies at the end of interval $E_1$, i.e. at the point of dissociation of the complex, the subsequent evolution of the free substrate with the BC, Anisochronous topology is the same.

The time-averaged amplitudes at the end of interval $E_2$ are given in Eq. 28. As discussed previously, the amplitude of the longitudinal two-spin order, $2\hat{R}_z\hat{T}_z$, does not evolve at all in the two-spin substrate at any field and is defined by its final value at the end of interval $E_1$.

The field-dependence of the $ZQ_x$ coherence in the Anisochronous model is shown in Fig. 10; between 0 mT and 20 mT in Fig. 10(a) and between 20 mT and 250 mT in Fig. 10(b). In contrast to the $2\hat{R}_z\hat{T}_z$ term , the $ZQ_x$ coherence does evolve. However, at small mixing fields, the chemical shift difference between the $^1$H nuclei on the free substrate is small, tending to zero at zero field. As can be seen from Eq. 32, when $\delta$ tends to zero the $ZQ_x$ amplitude tends to the value at the end of interval $E_1$. As the field increases, evolution due to the chemical shift difference becomes more significant, with a decrease in the amplitude as the mixing field increases. In the extreme case, where the sample has been transferred to the measurement field, the chemical shift difference is so large that the spin system may be described through the weak coupling approximation and the time average of the $ZQ_x$ amplitude tends to zero as described in the Time Averaging Section III D.

Fig. 11 illustrates the field-dependence of the longitudinal magnetisation in the Anisochronous model, from 0 - 20 mT, Fig 11(a) and 20 - 250 mT, Fig. 11(b). At low mixing field, < 12mT, $\delta$ tends to zero and the time-averaged amplitudes of $\hat{R}_z$ and $\hat{T}_z$ tend to the results for the Isochronous substrate, Eq. 32.

As the field increases, however, $b_z$, $c_z$, $d_z$ and $e_z$ from Eq. 31 tend to zero and the boundary amplitudes at the start of interval $E_2$, $a_{Rz}$ and $a_{Tz}$ tend to $f_z$ and $- f_z$, respectively, giving $\bar{c} = \dfrac{J\delta a_{ZQx} + \delta^2 f_z}{(J^2 + \delta^2)}$ and

$\bar{d} = \dfrac{-(J\delta a_{ZQx} + \delta^2 f_z)}{(J^2 + \delta^2)}$ as the time averaged-amplitudes for the terms $\hat{R}_z$ and $\hat{T}_z$ at the end of interval $E_2$. As explained previously, these terms do not evolve further during interval $E_3$. At the measurement field, the final time-averaged spin state of the Anisochronous substrate will be of the form $\bar{\rho} = a_{RzTz}2\hat{R}_z\hat{T}_z + \bar{c}\hat{R}_z + \bar{d}\hat{T}_z$.

The longitudinal magnetisation, $\hat{R}_z$ and $\hat{T}_z$, can then be read with a 90° pulse. As in the case of the Isochronous model, $2\hat{R}_z\hat{T}_z$, $\hat{R}_z$ and $\hat{T}_z$ can be read using a non-90° pulse. However, in the Anisochronous case, it is possible to choose a mixing field > 15mT at which the $\hat{R}_z\hat{T}_z$ term is not generated but at which $\hat{R}_z$ and $\hat{T}_z$ persist. In fact, for this particular model, 90 mT would give no $2\hat{R}_z\hat{T}_z$ contribution whilst maximising the contribution from $\hat{R}_z - \hat{T}_z$. This may be useful in MRI applications where an in-phase doublet may be advantageous.

**CONCLUSION**

The results presented herein demonstrate that scalar coupling between the /para/hydrogen and the substrate ^1 H nuclei in the complex provides a mechanism for polarisation transfer.

The precise state of the magnetisation reflects a fine balance between the scalar coupling and chemical shift effects and is field-dependent.

The models show that the substrate ^1 H nuclear spins can evolve into states which, when interrogated with a suitable RF-pulse could produce, in-phase doublets, originating from single spin Z-magnetisation, antiphase doublets originating from either two spin longitudinal order or residual zero quantum terms. Moreover under conditions where the chemical shift evolution is suppressed by use of a zero field, isochronous spins or even by spin locking, polarisation is predominantly transferred through spin pairs to produce long lived singlet states suitable for later interrogation.

**Claims**

1. A method of selective observation of non-hydrogenative *para*-hydrogen induced polarisation (NH-PHIP) in a material, as enhanced magnetic resonance signals, said method comprising the steps of:

   a) preparing a fluid containing spatially symmetric molecules comprising two halves each, with a non-Boltzmann nuclear spin state distribution of the symmetric molecules at the temperature of the fluid;
   b) providing a compound with a defined chemical identity;
   c) providing a template which binds or supports an interaction with parahydrogen and the compound and that offers sites of ordered environment for the two halves of the symmetric molecule and the compound which can be arranged at each site, wherein the ordered environment distinguishes chemically or magnetically the two halves of the symmetric molecule arranged at each site, and wherein the ordered environment allows interaction via scalar coupling or dipolar coupling between the two halves of the symmetric molecule and the compound arranged at each site; said template being selected from the group consisting of $[Ir(NHC)(H)_2(Py)_3]^+$, in which NHC is a saturated or unsaturated N-heterocyclic carbene; and analogues thereof;
   d) bringing together, at said temperature, the prepared fluid, the compound and the assembled template, thereby transferring the spin order from the symmetric molecules to the hyperpolarisable nuclei of the compound during a temporary association of the symmetric molecules, the compound at the template while ultimately keeping the chemical identity of the compound;
   e) performing an MR measurement on the compound comprising hyperpolarized nuclei prepared in step d); and
   f) separating thermal and longitudinal spin order states by suppressing or filtering a thermal magnetisation background signal created due to the interaction with a magnetic field of an instrument that is used, by the use of appropriate pulse sequences made up of appropriately phased RF pulses and receiver phases.

2. A method according to claim 1 wherein the method comprises observing the magnetic states generated through NH-PHIP by nuclear magnetic resonance (NMR) signals or rapid MRI measurements and wherein the thermal and longitudinal spin order states are separated.

3. A method according to claim 1 wherein the method comprises observing short lived states, e.g. for use in connection with nuclear magnetic resonance (NMR) signals or long lived states, e.g. for use in magnetic resonance imaging (MRI).

4. A method according to claim 1 wherein the method comprises observing long lived states created for pairs (or higher values) of coupled spins.

5. A method according to claim 4 wherein the pairs are hetero-nuclear in nature.

6. A method according to claim 5 wherein the hetero-nuclear pairs are selected from $^1H/^{13}C$, $^1H/^{19}F$ and $^1H/^{15}N$.

7. A method according to claim 4 wherein the pairs are homo-nuclear in nature.

8. A method according to claim 4 wherein the homo-nuclear pairs are selected from $^1H/^1H$, and $^{13}C/^{13}C$.

9. A method according to claim 1 wherein the separation according to step f) is achieved by

(i) separating the parahydrogen derived signals from those of the background exploiting differences that exist between the two types of magnetisation by using alternate addition and subtraction of $^1$H-NMR experiments;
(ii) use of a material that may be enriched in a coupled heteronucleus, and a series of gradient pulses, allowing the magnetisation to be selected based on the presence of the coupling to a second spin such as $^{13}$C or $^{31}$P, wherein the second spin acts as a filter, by producing a linked magnetic state, which can be read directly or transferred to another spin, where it is detected; or
(iii) the use of a multiple quantum or coherence based filter which selects *para*-hydrogen hyperpolarised signals by exploiting pulse field gradients to select a coherence transfer pathway that leads only to the detection of signals of interest (*Only Para-Hydrogen Spectroscop Y* (OPSY)).

10. A method according to claim 1 wherein the method comprises the use of a supported metal template such as $[Ir(IMes)(H)_2(Py)_3]^+$.

11. A method according to claim 1 wherein the hyperpolarisable nuclei <u>are selected from the group consisting of</u> H, D, $^{29}$Si, $^{13}$C, $^{15}$N, $^{31}$P and $^{19}$F.

12. A method according to claim 1 wherein the compound to be polarised is a metabolite.

13. A method according to claim 1 wherein the compound is subsequently used as a probe in an MRI experiment.

**Patentansprüche**

1. Verfahren zur selektiven Beobachtung einer nicht hydrierenden, *Para*wasserstoff induzierten Polarisation (non-hydrogenative para-hydrogen induced polarization = NH-PHIP) in ein Material, als verstärkte Magnetresonanzsignale, wobei das Verfahren die folgenden Schritte beinhaltet:

(a) Vorbereiten einer Flüssigkeit, die räumlich symmetrische Moleküle enthält, beinhaltend jeweils zwei Hälften, mit einer Nicht-Boltzmann-Kernspinzustand-Verteilung der symmetrischen Moleküle bei der Temperatur der Flüssigkeit;
(b) Vorbereiten einer Verbindung mit einer definierten chemischen Identität;
(c) Bereitstellen einer Vorlage, die eine Interaktion mit Parawasserstoff und der Verbindung bindet oder unterstützt und die Stellen einer geordneten Umgebung für die zwei Hälften des symmetrischen Moleküls und die Verbindung bietet, die an jeder Stelle angeordnet werden können, und wobei die geordnete Umgebung Interaktion über skalares Koppeln oder dipolares Koppeln zwischen den zwei Hälften des symmetrischen Moleküls und der Verbindung, die an jeder Stelle angeordnet sind, erlaubt; wobei die Vorlage aus der Gruppe selektiert ist, die aus $[Ir(NHC)(H)_2(Py)_3]^+$ besteht, bei der NHC ein gesättigtes und ungesättigtes N-heterocyclisches Carben und Analoga davon ist;
(d) Zusammenbringen, bei der Temperatur, der vorbereiteten Flüssigkeit, der Verbindung und der zusammengesetzten Vorlage, wodurch die Spinordnung von den symmetrischen Molekülen auf die hyperpolarisierbaren Nuklei der Verbindung während einer vorübergehenden Assoziation der symmetrischen Moleküle transferiert wird, der Verbindung der Vorlage, während letztendlich die chemische Identität der Verbindung erhalten bleibt;
(e) Durchführen einer MR-Messung an der Verbindung, die hyperpolarisierte Nuklei beinhaltet, die in Schritt d) vorbereitet wurden; und
(f) Trennen der thermischen und longitudinalen Spinordnungszustände durch Unterdrücken oder Filtern eines thermischen Magnetisierungshintergrundsignals, das aufgrund der Interaktion mit einem Magnetfeld eines Instruments, das verwendet wird, erzeugt wird, durch die Verwendung von entsprechenden Impulsfolgen, die sich aus entsprechend phasenverschobenen HF-Impulsen und Empfängerphasen zusammensetzen.

2. Verfahren gemäß Anspruch 1, wobei das Verfahren das Beobachten der erzeugten magnetischen Zustände beinhaltet, durch NH-PHIP, mittels kernmagnetische Resonanzsignale (NMR-Signale) oder schnelle MRI Messungen, und wobei die thermischen und longitudinalen Spinordnungszustände getrennt sind.

3. Verfahren gemäß Anspruch 1, wobei das Verfahren das Beobachten kurzlebiger Zustände, z. B. zur Verwendung in Verbindung mit kernmagnetischen Resonanzsignalen (NMR-Signalen), oder langlebiger Zustände, z. B. zur Verwendung bei Magnetresonanzbildgebung (MRI), beinhaltet.

4. Verfahren gemäß Anspruch 1, wobei das Verfahren das Beobachten langlebiger Zustände, die für Paare (oder höhere Werte) von gekoppelten Spins erzeugt wurden, beinhaltet.

5. Verfahren gemäß Anspruch 4, wobei die Paare heteronuklear beschaffen sind.

6. Verfahren gemäß Anspruch 5, wobei die heteronuklearen Paare aus $^1$H/$^{13}$C, $^1$H/$^{19}$F und $^1$H/$^{15}$N selektiert sind.

7. Verfahren gemäß Anspruch 4, wobei die Paare homonuklear beschaffen sind.

8. Verfahren gemäß Anspruch 4, wobei die homonuklearen Paare aus $^1$H/$^1$H, und $^{13}$C/$^{13}$C selektiert sind.

9. Verfahren gemäß Anspruch 1, wobei die Trennung gemäß Schritt f) erreicht wird durch

(i) Trennen der Parawasserstoff abgeleiteten Signale von jenen des Hintergrunds durch Nutzen der Differenzen, die zwischen den zwei Arten der Magnetisierung bestehen, durch Verwenden alternierender Addition und Subtraktion von $^1$H-NMR-Experimenten;
(ii) Verwendung eines Materials, das in einem gekoppelten Heteronukleus angereichert sein kann, und einer Reihe von Gradientimpulsen, wodurch die Magnetisierung basierend auf der Präsenz des Koppelns eines zweiten Spins, wie etwa $^{13}$C oder $^{31}$P, selektiert werden kann, wobei der zweite Spin als Filter wirkt, durch Produzieren eines verknüpften magnetischen Zustands, der direkt gelesen oder auf einen anderen Spin transferiert werden kann, wo er detektiert wird; oder
(iii) Verwendung eines Mehrfachquanten oder Kohärenz basierten Filters, der Parawasserstoff hyperpolarisierte Signale durch Nutzen der Impulsfeldgradienten selektiert, um einen Kohärenztransferweg zu selektieren, der nur zur Detektion von Signalen von Interesse führt *(Only Para-Hydrogen SpectroscopY* (OPSY)).

10. Verfahren gemäß Anspruch 1, wobei das Verfahren die Verwendung einer unterstützten Metallvorlage, wie etwa [Ir(IMes)(H)$_2$(P$_y$)$_3$]$^+$, beinhaltet.

11. Verfahren gemäß Anspruch 1, wobei die hyperpolarisierbaren Nuklei aus der Gruppe selektiert sind, die aus H, D, $^{29}$Si, $^{13}$C, $^{15}$N, $^{31}$P und $^{19}$F besteht.

12. Verfahren gemäß Anspruch 1, wobei die zu polarisierende Verbindung ein Metabolit ist.

13. Verfahren gemäß Anspruch 1, wobei die Verbindung anschließlich als Sonde in einem MRI-Experiment verwendet wird.

**Revendications**

1. Procédé d'observation sélective d'une polarisation induite par *para*-hydrogène non hydrogénante (NH-PHIP) dans un matériau, comme signaux de résonance magnétique renforcés, ledit procédé comprenant les étapes de :

a) préparation d'un fluide contenant des molécules symétriques spatialement comprenant deux moitiés chacune, avec une distribution d'état de spin nucléaire non de Boltzmann des molécules symétriques à la température du fluide ;
b) obtention d'un composé dont l'identité chimique est définie ;
c) obtention d'un modèle qui lie ou supporte une interaction avec le para-hydrogène et le composé et qui offre des sites d'environnement ordonné pour les deux moitiés de la molécule symétrique et le composé qui peuvent être disposés à chaque site, et ledit environnement ordonné permettant une interaction par l'intermédiaire d'un couplage scalaire ou d'un couplage dipolaire entre les deux moitiés de la molécule symétrique et le composé disposés à chaque site ; ledit modèle étant sélectionné dans le groupe constitué par [Ir(NHC)(H)$_2$(Py)$_3$]$^+$, NHC étant un carbène N-hétérocyclique insaturé ou saturé, et ses analogues ;
d) mélange, à la dite température, du fluide préparé, du composé et du modèle assemblé, transférant ainsi l'ordre de spin provenant des molécules symétriques aux noyaux hyper-polarisables du composé pendant une association temporaire des molécules symétriques, du composé au niveau du modèle tout en maintenant à la fin l'identité chimique du composé ;
e) réalisation d'une mesure RM sur le composé comprenant les noyaux hyper-polarisés préparés à l'étape d) ; et
f) séparation des états d'ordre de spin thermiques et longitudinaux par suppression ou filtrage d'un signal d'arrière-plan de magnétisation thermique créé de par l'interaction avec un champ magnétique d'un instrument qui est utilisé, à l'aide de séquences d'impulsions appropriées constituées d'impulsions RF synchronisées de manière appropriée et de phases de réception.

2. Procédé selon la revendication 1, ledit procédé comprenant l'observation des états magnétiques générés par NH-PHIP par des signaux de résonance magnétique nucléaire (RMN) ou des mesures par IRM rapides et lesdits états d'ordre de spin longitudinaux et thermiques étant distincts.

3. Procédé selon la revendication 1, ledit procédé comprenant l'observation d'états de courte durée de vie, destinés, par exemple, à être utilisés en relation avec des signaux de résonance magnétique nucléaire (RMN) ou des états de longue durée de vie, destinés, par exemple, à être utilisés en imagerie par résonance magnétique (IRM).

4. Procédé selon la revendication 1, ledit procédé comprenant l'observation d'états de longue durée de vie créés pour des paires (ou des valeurs plus élevées) de spins couplés.

5. Procédé selon la revendication 4, lesdites paires étant hétéro-nucléaires par nature.

6. Procédé selon la revendication 5, lesdites paires hétéro-nucléaires étant sélectionnées parmi $^1$H/$^{13}$C, $^1$H/$^{19}$F et $^1$H/$^{15}$N.

7. Procédé selon la revendication 4, lesdites paires étant homo-nucléaires par nature.

8. Procédé selon la revendication 4, lesdites paires homo-nucléaires étant sélectionnées parmi $^1$H/$^1$H et $^{13}$C/$^{13}$C.

9. Procédé selon la revendication 1, ladite séparation selon l'étape f) étant accomplie par

(i) séparation des signaux dérivés du para-hydrogène de ceux de l'arrière-plan en exploitant les différences qui existent entre les deux types de magnétisation à l'aide de l'alternance d'addition et de soustraction d'expériences de $^1$H-RMN ;
(ii) utilisation d'un matériau qui pourrait être enrichi dans un hétéro-noyau couplé, et d'une série d'impulsions de gradient, permettant à la magnétisation d'être sélectionnée sur la base de la présence du couplage à un second spin tel que $^{13}$C ou $^{31}$P, ledit second spin agissant comme un filtre, en produisant un état magnétique lié, qui peut être lu directement ou transféré à un autre spin, où il est détecté ; ou
(iii) utilisation d'un filtre quantique multiple ou d'un filtre basé sur la cohérence qui sélectionne les signaux hyper-polarisés par *para*-hydrogène par l'exploitation de gradients de champ d'impulsions pour sélectionner un trajet de transfert de cohérence qui conduit seulement à la détection de signaux d'intérêt (S*pectroscopie* OPSY (spectroscopie du para-hydrogène uniquement)).

10. Procédé selon la revendication 1, ledit procédé comprenant l'utilisation d'un modèle de métal supporté tel que [Ir(IMeS)(H)$_2$(Py)$_3$]$^+$.

11. Procédé selon la revendication 1, lesdits noyaux hyper-polarisables étant sélectionnés dans le groupe constitué par H, D, $^{29}$Si, $^{13}$C, $^{15}$N, $^{31}$P et $^{19}$F.

12. Procédé selon la revendication 1, ledit composé à polariser étant un métabolite.

13. Procédé selon la revendication 1, ledit composé étant par la suite utilisé comme une sonde dans une expérience d'IRM.

vertical expansion * 32

(a) 9.5 9.0 8.5 8.0 7.5 7.0 ppm

(b) 145 140 135 130 125 120 ppm

(c) 4 2 0 -2 -4

(d) 9.4 9.0 8.6 8.2 7.8 7.4 ppm

(e) 170 160 150 140 130 120

**Figure 1**

(a)

(b)

**Figure 2**

(a)

(b)

(c)

(d)

**Figure 3**

(a)

(b)

**Figure 5**

**Figure 6**

**sScheme 1.**

**sFig. 1.**

sFig. 2.

sFig. 3.

sFig. 4.

sFig. 6.

sFig. 7.

FIG. 2.

FIG. 3.

Mixing Field (mT)

FIG. 4.

FIG. 5.

FIG. 6.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008155093 A
- GB 0822484 A
- WO 9924080 A
- EP 1047455 A
- US 6574495 B
- US 20040024307 A
- WO 2004019995 A
- US 7459144 B
- WO 9935508 A
- EP 1046051 A
- US 6311086 B

- US 6466814 B
- US 6278893 B
- WO 0223210 A
- WO 0223209 A
- WO 2005015253 A
- WO 0196895 A
- US 20060003926 A
- WO 2004048988 A
- WO 2007136439 A
- WO 2007082048 A

**Non-patent literature cited in the description**

- **BLAZINA et al.** *Dalton Trans.,* 2004, 2601-2609
- **KEMSLEY.** *Chemical and Engineering News,* 2008, vol. 86, 12-15
- *Coordination Chemistry reviews,* 2008, vol. 252, 2278-2291
- **BHATTACHARYA et al.** Ultra-fast three dimensional imaging of hyperpolarized 13C in vivo. *MAGMA,* 2005, vol. 18, 245-256

- **BOUCHARD L.-S. et al.** *Angew. Chem. Int. Ed.,* 2007, vol. 46, 4064-4068
- Remington's Pharmaceutical Sciences. Mack Publishing Co, 1975, 1405-1412, 1461-1487
- The National Formulary XIV. American Pharmaceutical Association, 1975